# EUROPEAN PATENT SPECIFICATION

(11) **EP 3 176 583 B1**
(45) Date of publication and mention of the grant of the patent: **11.09.2019**
(21) Application number: 16200905.4
(22) Date of filing: 13.06.2012
(51) Int. Cl.: G01N 33/68, G01N 33/50, C07K 14/52, C07K 16/00, A61M 1/36, B01J 20/32

(54) **DIAGNOSING AND TREATING INFLAMMATORY BOWEL DISEASE AND IRRITABLE BOWEL SYNDROME**
DIAGNOSE UND BEHANDLUNG VON ENTZÜNDLICHER DARMERKRANKUNG UND REIZDARMSYNDROM
DIAGNOSTIC ET TRAITEMENT DE MALADIE INTESTINALE INFLAMMATOIRE ET DU SYNDROME DU CÔLON IRRITABLE

(30) Priority: 13.06.2011 US 201161496442 P
(43) Date of publication of application: 07.06.2017
(62) Divisional of application: 12727680.6
(73) Proprietor: TLA Targeted Immunotherapies AB, 171 76 Stockholm (SE)
(72) Inventor: WINQVIST, Ola, 756 53 Uppsala (SE); COTTON, Graham, Edinburgh Central Scotland EH13 9PH (GB)
(74) Representative: Zacco Denmark A/S

(56) References cited:
- EP-A2- 1 255 112
- WO-A1-2008/142405
- WO-A1-2011/017120
- WO-A2-2010/029317
- AUTSCHBACH F ET AL: "Expression of chemokine receptors in normal and inflamed human intestine, tonsil and liver", CELLULAR IMMUNOLOGY, vol. 236, 23 September 2005 (2005-09-23), pages 110-114, XP002682030,
- M. J. WALTERS ET AL: "Characterization of CCX282-B, an Orally Bioavailable Antagonist of the CCR9 Chemokine Receptor, for Treatment of Inflammatory Bowel Disease", JOURNAL OF PHARMACOLOGY AND EXPERIMENTAL THERAPEUTICS, vol. 335, no. 1, 1 October 2010 (2010-10-01), pages 61-69, XP055035781, ISSN: 0022-3565, DOI: 10.1124/jpet.110.169714
- H. HANAI ET AL: "The mode of actions of the Adacolumn therapeutic leucocytapheresis in patients with inflammatory bowel disease: a concise review", CLINICAL & EXPERIMENTAL IMMUNOLOGY, vol. 163, no. 1, 16 November 2010 (2010-11-16), pages 50-58, XP055035673, ISSN: 0009-9104, DOI: 10.1111/j.1365-2249.2010.04279.x
- LUDVIG LINTON ET AL: "CCR9-expressing CD14+HLA-DRhi blood monocytes promote intestinal inflammation in IBD", JOURNAL OF TRANSLATIONAL MEDICINE, vol. 9, no. Suppl 2, 23 November 2011 (2011-11-23), page P32, XP055035715, ISSN: 1479-5876, DOI: 10.1186/1479-5876-9-S2-P32
- LUDVIG LINTON ET AL: "HLA-DRhi and CCR9 Define a Pro-Inflammatory Monocyte Subset in IBD", CLINICAL AND TRANSLATIONAL GASTROENTEROLOGY, vol. 3, no. 12, 20 December 2012 (2012-12-20), page e29, XP055362554, DOI: 10.1038/ctg.2012.23

## Description

### FIELD OF THE INVENTION

The various embodiments of the present invention relate to products for and methods of treating inflammatory conditions, such as inflammatory bowel disease, including Crohn's disease (CD) and ulcerative colitis (UC). Companion diagnostics are also described.

### BACKGROUND OF THE INVENTION

Fulminant ulcerative colitis is a worsening of ulcerative colitis characterized by a high white blood cell count and severe abdominal pain. At present, patients with fulminant ulcerative colitis are treated with high doses of steroids. In phase III-studies treatment with anti-TNFa has been investigated. Both drugs are general inhibitors of inflammation. They are effective in about 50% of cases but have serious adverse effects. Even if successfully treated fulminant ulcerative colitis has a tendency of recurring.

In patients with fulminant ulcerative colitis not responding to medical treatment prompt surgical intervention is mandatory. Ulcerative colitis is always restricted to the large intestine (colon). As a last measure the colon is resected, and an external ileostoma constructed. After a recovery period of at least 6 months and sometimes further medical treatment of rectal stump inflammation either ileorectal anastomosis or reconstructive surgery with a pelvic pouch will be performed in most patients to restore intestinal continuity. Both procedures entail loose stools about six times daily and disturbances in water- and mineral balances. There may also be fulminant episodes in Crohn's disease (fulminant Crohn's colitis), which are also serious conditions necessitating immediate medical and/or surgical intervention.

While the inflammation can be located in any part of the gastrointestinal tract in patients with Crohn's disease, it is usually confined to the most distal part of the small intestine and the first part of the large intestine (ileocaecal region). Medical treatment cannot cure the disease although anti-inflammatory drugs such as steroids and aza-thioprine relieve symptoms. Surgery with resection of stenotic and fistulating bowel segments is indicated in about 50% of patients; half of them will have recurrences and need further surgery. A method which can specifically turn off the inflammation in IBD and prevent recurrent disease in the individual patient thus is highly warranted.

Apheresis is a treatment used for depletion of blood components, such as antibodies, low-density lipoproteins (LDL) and blood cells. Leukapheresis is the apheresis treatment used for removal of white blood cells, leukocytes. The patient is connected to an extracorporeal blood circulating system; the blood is drawn from a vein in one arm, passed through a column device and returned into the other arm of the patient. WO2010/029317 describes apheresis columns useful for treating inflammatory conditions including a chemokine immobilised on a solid support.

Autschbach, F et al., Cellular Immunology, 2005, 236, pp. 110-114, relates to chemokines as important mediators involved in the recruitment of leucocytes to lymphoid tissues and to sites of inflammation, identifying suitable monoclonal antibodies for immunohistochemistry, by a tested a panel of new reagents provided by the cytokine/chemokine receptor section of the 8th HLDA workshop on cryostat sections of normal human gut, tonsil, and liver. In addition, inflamed intestinal tissues from individuals with Crohn's disease and ulcerative colitis were analysed. Results reveal an upregulated expression of chemokine receptors like CCR6, CCR7, CCR9, CXCR1, and CXCR3 in inflamed gut.

Matthew, J. W., et al., J. Pharmacol. Experimental Ther., 2010, 335(1), pp. 61-69, relates to the discovery of a small molecule, CCX282-B, which is an orally bioavailable, selective, and potent antagonist of human CCR9. CCX282-B inhibited CCR9-mediated Ca(2+) mobilization and chemotaxis on Molt-4 cells with IC(50) values of 5.4 and 3.4 nM, respectively. In the presence of 100% human serum, CCX282-B inhibited CCR9-mediated chemotaxis with an IC(50) of 33 nM, and the addition of α1-acid glycoprotein did not affect its potency. CCX282-B inhibited chemotaxis of primary CCR9-expressing cells to CCL25 with an IC(50) of 6.8 nM. CCX282-B was an equipotent inhibitor of CCL25-directed chemotaxis of both splice forms of CCR9 (CCR9A and CCR9B) with IC(50) values of 2.8 and 2.6 nM, respectively. CCX282-B also inhibited mouse and rat CCR9-mediated chemotaxis. Inhibition of CCR9 with CCX282-B results in normalization of Crohn's disease such as histopathology associated with the TNF(ΔARE) mice.

### SUMMARY OF THE INVENTION

Chemokines are a class of cytokine molecules involved in cell recruitment and activation in inflammation. Chemokines cause chemotaxis and activation of various subpopulations of cells in the immune system. The activity of chemokines is mediated primarily through tight binding to their receptors on the surface of leukocytes. In certain embodiments the present invention is based on the realisation that the interaction between (specific) chemokines and (specific) cells expressing their receptors may be exploited for the treatment of inflammatory bowel disease, including Crohn's disease (CD) and ulcerative cholitis (UC). The inventors have determined that targeting increased recruitment of specific chemokine receptor-expressing cells to the site of inflammation presents a new therapeutic approach to treat such conditions. Moreover, in such conditions, chemokine receptor expression on each cell may be increased again providing a therapeutic approach to treat such conditions.

The inventors have also determined that the frequency of circulating CD14+HLA-DRhi monocytes was significantly higher in IBD patients with moderate-to-severe disease compared to healthy controls. Furthermore, the presence of these monocytes correlated significantly to disease activity in patients with ulcerative colitis and Crohn's disease. This provides a further target for treatment of IBD which may be utilised in combination with targeting cells expressing chemokine receptors. This population of monocytes expresses high levels of CCR7 and CCR9.

Thus, present invention relates to a binding reagent capable of specifically binding to a marker of CD14+HLA-DRhi monocytes selected from CCR9 for use in the treatment of inflammatory bowel disease, wherein the binding reagent is immobilized on a solid support contained within an apheresis column, to which is applied peripheral blood from a patient thus removing CD14+HLA-DRhi monocytes from the peripheral blood of the patient or subject, and wherein the binding agent is an antibody.

Moreover, present invention also relates to a method of diagnosing, monitoring progression of, or monitoring treatment of inflammatory bowel disease comprising determining the levels of monocytes expressing CCR9 in a sample obtained from a subject, wherein high levels of monocytes expressing CCR9, or increased levels of monocytes expressing CCR9 compared to the levels of monocytes expressing CCR9 in a healthy subject, indicate the presence or progression of inflammatory bowel disease, and wherein the subject has been treated using a binding agent according to the invention.

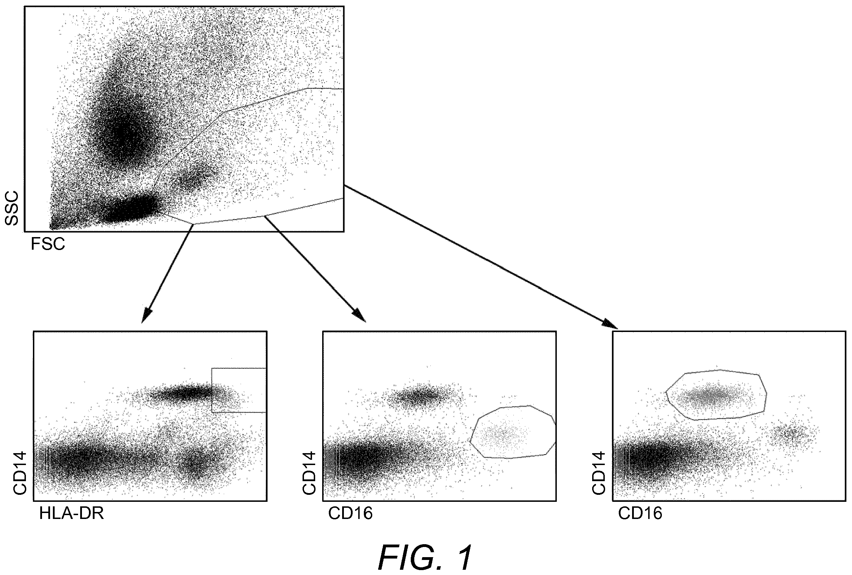

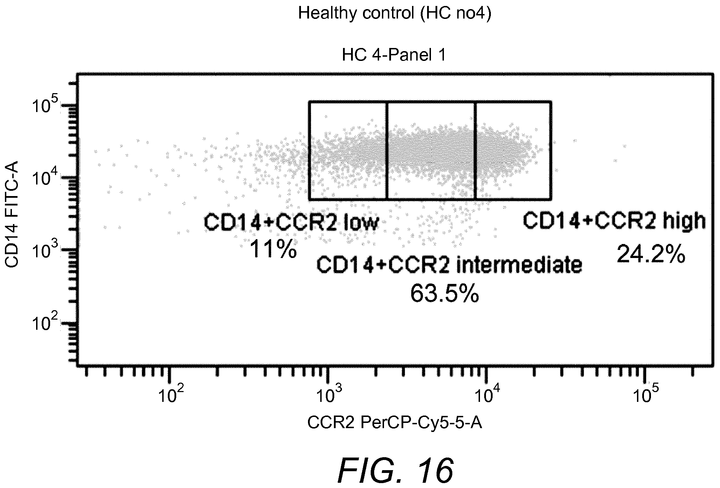

As shown herein, suitable binding reagents can be immobilized onto a solid support, either directly or indirectly, to generate an apheresis column suitable for capturing relevant receptor-expressing cells. Where increased levels of receptor expression are observed, such cells may be preferably removed from the peripheral blood using the columns of the various embodiments of the invention. Thus, the methods of various embodiments of the invention targeting CD14+HLA-DRhi cells as defined herein for removal from the peripheral blood. "High" expression may be determined according to standard flow cytometry techniques, as discussed in further detail herein. An example is shown in Fig.1 and Fig. 16 herein of a gating strategy. HLA-DR is a MHC class II cell surface receptor encoded by the human leukocyte antigen complex on chromosome 6 region 6p21.31.

The inventors have also surprisingly found that Irritable Bowel Syndrome (IBS) patients, or subject suffering from IBS, display an increased frequency (or level) of chemokine receptor expressing cells, in particular monocytes, more specifically CCR9 expressing monocytes. Thus, IBS patients may display inflammation that is comparable to that shown by patients suffering from IBD (levels of CCR9 expression may be similar to those found in UC and CD as discussed herein). Irritable bowel syndrome (IBS) is a condition characterized by chronic abdominal pain, discomfort, bloating, and alteration of bowel habits. It is currently diagnosed on the basis of symptoms only. Accordingly, identification of a pro-inflammatory component provides new avenues for treatment and diagnosis of this debilitating condition. In particular, it is shown herein that the CCR9 expressing cells increased in IBS patients can be depleted using a leukapheresis approach and/or a monocyte reduction therapy.

Thus, in certain embodiments the invention serves to reduce the recruitment of inflammatory leukocytes, which express characteristic receptors, including chemokine receptors, and possibly express characteristic receptors, in particular chemokine receptors at increased levels, to sites of inflammation linked to IBS. This is achieved using specific binding reagents to capture specific receptor-expressing inflammatory leukocytes from the patient. Accordingly, the disclosure enables a method for treating IBS comprising applying peripheral blood from a patient to an apheresis column loaded with a solid support comprising one or more binding reagents capable of specifically binding to chemokine receptor expressing cells, in particular CCR9 expressing cells immobilized directly or indirectly on the support thus removing the chemokine receptor expressing cells from the peripheral blood of the patient. The peripheral blood from which the chemokine receptor expressing cells have been removed may then be returned to the patient in order to complete the treatment. In certain embodiments the invention may thus rely on a continuous extracorporeal circuit in some embodiments. Alternatively, in certain embodiments the invention may comprise steps of obtaining peripheral blood from the patient, applying the peripheral blood to the column and subsequently returning the peripheral blood from which the chemokine receptor expressing cells have been removed to the patient.

As shown herein, suitable binding reagents can be immobilized onto a solid support, either directly or indirectly, to generate an apheresis column suitable for capturing relevant receptor-expressing cells. Where increased levels of receptor expression are observed, such cells may be preferably removed from the peripheral blood using the columns of the various embodiments of the disclosure. Thus, the methods of various embodiments of the invention preferably target "CCR9hi" expressing cells as defined herein for removal from the peripheral blood. "High" expression may be determined according to standard flow cytometry techniques, as discussed in further detail herein. An example is shown in Fig.1 and Fig. 16 herein of a gating strategy.

In other embodiments the invention further provides a binding reagent capable of specifically binding to a marker of CD14+HLA-DRhi monocytes selected from CCR9 for use in the treatment of inflammatory bowel disease, including Crohn's disease (CD) and ulcerative cholitis (UC), wherein the binding reagent is immobilized, directly or indirectly, on a solid support contained within an apheresis column, to which is applied peripheral blood from a patient thus removing CD14+HLA-DRhi monocytes from the peripheral blood of the patient. In certain embodiments the invention also provides for use of a binding reagent capable of specifically binding to a marker of CD14+HLA-DRhi monocytes selected from CCR9 for use in the manufacture of an apheresis column for treatment of inflammatory bowel disease, including Crohn's disease (CD) and ulcerative cholitis (UC), wherein the one or more binding reagents is immobilized on a solid support contained within an apheresis column, to which is applied peripheral blood from a patient thus removing CD14+HLA-DRhi monocytes from the peripheral blood of the patient.

In certain parts, the disclosure enables treatment of inflammatory bowel disease, including Crohn's disease (CD) and ulcerative cholitis (UC). By treatment is meant a reduction in the specific receptor, including chemokine receptor, expressing cells in the peripheral blood of the patient. The reduction may comprise a reduction in cells that express receptors, including chemokine receptors, in particular CCR9, at increased levels in diseased patients. The patient is typically a human patient but the term patient may include both human and non-human animal subjects in some embodiments.

In other parts the disclosure aims to treat IBS. By treatment is meant a reduction in the specific receptor, including chemokine receptor, expressing cells in the peripheral blood of the patient. The reduction comprise a reduction in cells that express receptors, i.e. the CCR9receptor, at increased levels in diseased patients. The patient is typically a human patient but the term patient may include both human and non-human animal subjects in some embodiments. In the context of the various embodiments of the present invention, this typically involves a reduction in CCR9 expressing cells, CCR9^{hi} expressing cells, in the peripheral blood of the patient. The CCR9 expressing cells comprise, consist essentially of or consist of monocytes, in particular CD14+HLA-DR^{hi} monocytes, in certain embodiments. This cell type can be characterized according to the techniques disclosed herein (for example using flow cytometry).

Monocytes are produced by the bone marrow from haematopoietic stem cell precursors called monoblasts. Monocytes may differentiate into macrophages or dendritic cells. Monocytes and their macrophage and dendritic cell progeny serve a number of functions in the immune system including phagocytosis, antigen presentation and cytokine production. Monocytes may be characterized with reference to expression of the cell surface marker CD14, optionally together with CD16. Classical monocytes may be characterized by high level expression of the CD14 cell surface receptor (CD14++ CD16- monocyte). Non-classical monocytes may be characterized by low level expression of CD14 and with additional co-expression of the CD16 receptor (CD14+CD16++ monocyte). Intermediate monocytes may be characterized by high level expression of CD14 and low level expression of CD16 (CD14++CD16+ monocytes). Macrophages are derived from monocytes and are responsible for protecting tissues from foreign substances. They are cells that possess a large smooth nucleus, a large area of cytoplasm and internal vesicles for processing foreign material. The term "macrophage" may refer to a monocyte-derived cell expressing one or more of the following cell surface markers CD14, CD11b, Lysozyme M, MAC-1/MAC-3 and CD68. The term macrophage includes both activated and un-activated macrophages. Activated macrophages may be characterized by expression of CD69, ENG, FCER2 and IL2RA, HLA-DR, CD86. Un-activated macrophages have not yet received activating signals through for example TLR receptors and therefore they express less activation markers on the cell surface which correlates with lesser maturation. M1 macrophages may be characterized by expression of one or more of CD16⁺CD32⁺CD64⁺ and secrete mainly IL-23 and IL-1, TNF, IL-6 and high levels of IL-12 and in addition effector molecules such as iNOS and ROI. M1 macrophages have cytotoxic features as opposed to M2 macrophages. M2 macrophages may be characterized by expression of one or more of SRA/B⁺CD163⁺MR⁺CD14⁺ and express TGFβ, IL-10 and IL-1Ra. Tumour associated macrophages (TAMs) share many characteristics with the M2 macrophages and are considered as M2 polarised macrophages. The M1/M2 paradigm can also be found in monocyte subsets where CD14⁺CD16⁻CXC3R1^{low} monocytes are considered the "inflammatory" subset and the CD14^{low}CD16⁺CXC3R1^{high} are "resident" monocytes.

CCR9 expressed on these aforementioned cells binds to chemokines such as CCL25. CCR9 is the gene symbol approved by the HUGO Gene Nomenclature Committee for chemokine (C-C motif) receptor 9. The HGNC ID for this gene is 1610. The gene is located at chromosome position 3p22. The previous symbol and name for the gene is GPR28. Synonyms for this gene include CDw199, GPR-9-6. The Genbank reference sequence for CCR9 is AJ132337.1 as available on 13 June 2011.

Treatment of IBD according to the various disclosures may result in alleviation or amelioration of symptoms, prevention of progression, regression of the condition, or complete recovery. Measurable parameters of successful treatment include one or more, up to all, of improved Mayo score or UCDAI (for UC) or improvement in CDAI index (CD). In specific embodiments, a single treatment is sufficient to cause a depletion of around 10%, 20%, 30%, 40%, 50%, 60% or 70%, or higher up to 80%, 90%, 95% or more, or any range of values between and including these amounts, of a specific receptor, including CCR9 expressing cells, in particular CD14+HLA-DRhi monocytes from the peripheral blood of the patient. In specific parts of the disclosure, at least around 50% depletion is achieved in a single treatment. Thus, successful treatment may be defined with reference to depletion of CD14+HLA-DR^{hi} monocytes. Treatment may lead to depletion of between approximately 100 and 500 million CD14+HLA-DR^{hi} monocytes.

Treatment of IBS according to the disclosure may result in alleviation or amelioration of symptoms, prevention of progression, regression of the condition, or complete recovery. In specific embodiments, a single treatment is sufficient to cause a depletion of around 10%, 20%, 30%, 40%, 50%, 60% or 70%, or higher up to 80%, 90%, 95% or more, or any range of values between and including these amounts, of CCR9 expressing cells, in particular CCR9 expressing monocytes from the peripheral blood of the patient. In specific parts of the disclosure, at least around 50% depletion is achieved in a single treatment. Thus, successful treatment may be defined with reference to depletion of CCR9 expressing cells, in particular CCR9 expressing monocytes, such as CD14+HLA-DR^{hi} monocytes. Treatment may lead to depletion of between approximately 100 and 500 million CCR9 expressing cells, in particular CCR9 expressing monocytes, such as CD14+HLA-DR^{hi} monocytes, in certain embodiments and more particularly to about 100, 150, 200, 250, 300, 350, 400, 450, or 500 million CCR9 expressing cells.

By binding to the column through the binding reagent-receptor, including chemokine receptor, interaction, receptor, including chemokine receptor, expressing cells are immobilized. These immobilized cells express further unoccupied chemokine receptors, which may be of the same or different type to those used for capture. These additional chemokine receptors may permit circulating chemokines which contribute to the inflammatory condition to be captured from the peripheral blood. Thus, a reduction in circulating (specific) chemokine levels may provide a measure of successful treatment. By "specific binding" is meant that the binding reagent displays sufficient specificity of binding and appropriate binding affinity/kinetics to permit removal of cells expressing CCR9, and specifically CCR9 in the case of IBS in particular CD14+HLA-DR^{hi} monocytes from the peripheral blood.

It is specifically envisaged that combinations of binding reagents may be employed in order to deplete levels of CD14⁺HLA-DR^{hi} monocytes or monocytes expressing CCR9 from peripheral blood.

Antibodies against the relevant chemokine receptor are generally considered to be antagonists, as would be appreciated by one skilled in the art. Specific binding reagents include antibodies.

In the context of the disclosure the term "chemokine" also comprises biotinylated or otherwise labelled chemokines. The term "chemokine" also comprises modified and truncated versions of the chemokine and chemokine fragments with the proviso that the modified or truncated form retains its ability to bind to its cognate receptor (and thus remains functional in the context of the various embodiments of the invention). The chemokine does not necessarily need to retain biological activity as it is specific binding affinity for CCR9 and/or CCR7 that is required. In certain embodiments, the chemokine lacks biological activity, for example in terms of activation of the (CCR9 and/or CCR7) receptor. CCL25 is the gene symbol approved by the HUGO Gene Nomenclature Committee for chemokine (C-C motif) ligand 25. The HGNC ID for this gene is 10624. The gene is located at chromosome position 19p13.2. The previous symbol and name for the gene is SCYA25, "small inducible cytokine subfamily A (Cys-Cys), member 25". Synonyms for this gene include "Ck beta-15", Ckb15, TECK, "TECKvar", "thymus expressed chemokine". The Genbank reference sequence for CCL25 is U86358.1 as available on 13 June 2011.

CCL21 is the gene symbol approved by the HUGO Gene Nomenclature Committee for chemokine (C-C motif) ligand 21. The HGNC ID for this gene is 10620. The gene is located at chromosome position 9p13. The previous symbol and name for the gene is SCYA21, "small inducible cytokine subfamily A (Cys-Cys), member 21". Synonyms for this gene include 6Ckine, "beta chemokine exodus-2", CKb9, ECL, "Efficient Chemoattractant for Lymphocytes", exodus-2, "secondary lymphoid tissue chemokine", SLC, TCA4. The Genbank reference sequence for CCL21 is AB002409.1 as available on 13 June 2011.

CCL19 is the gene symbol approved by the HUGO Gene Nomenclature Committee for chemokine (C-C motif) ligand 19, also known as MIP-3b. The HGNC ID for this gene is 10617. The gene is located at chromosome position 9p13. The previous symbol and name for the gene is SCYA19, "small inducible cytokine subfamily A (Cys-Cys), member 19". Synonyms for this gene include "beta chemokine exodus-3", "CC chemokine ligand 19", "CK beta-11", CKb11, "EBI1-ligand chemokine", ELC, exodus-3, "macrophage inflammatory protein 3-beta", MIP-3b. The Genbank reference sequence for CCL19 is AB000887.1 as available on 13 June 2011.

An example of a chemokine of the disclosure containing both modifications and a truncation and specifically adapted for use in the invention is described in detail herein. The truncated CCL25 corresponds to residues 1 to 74 of the full length mature protein (and thus lacks amino acids 75 to 127 and the N-terminal signal peptide of 23 amino acids) and thus retains the chemokine fold. In addition, a methionine to Norleucine substitution is incorporated, to prevent oxidation of the residue during chain assembly. The N terminal glutamine residue is substituted with pyroglutamine to permit uniformity of product during synthesis. Biotinylation is achieved via a PEG spacer at the ε-functionatity of the lysine residue found at position 72. The amino acid sequence of the linear molecule (i.e. without the PEG spacer and biotin molecule at amino acid 72 shown) comprises, consists essentially of or consists of the amino acid sequence presented as SEQ ID NO: 1. The final protein may thus comprise, consist essentially of or consist of the amino acid sequence of SEQ ID NO: 3.

A further example of a chemokine of the disclosure containing modifications and specifically adapted for use in the invention is described in detail herein (see Example 9 below). The modified CCL19 (MIP-3β) corresponds to residues 1 to 77 of the full length mature protein (and lacks the N-terminal signal peptide of 21 amino acids, which is cleaved off) and thus retains the chemokine fold. An additional lysine is inserted at the C-terminus, at position 78. The chemokine may thus comprise, consist essentially of or consist of the amino acid sequence of SEQ ID NO: 4. FmocLys(ivDde)-OH is incorporated as residue 78 to facilitate site-specific labelling at this position of the protein (SEQ ID NO: 5). The ε-amino side chain functionality of Lys(78) is modified through biotinylation. The final protein may thus comprise, consist essentially of or consist of the amino acid sequence of SEQ ID NO: 6.

Thus, in present disclosure a modified chemokine comprising, consisting essentially of or consisting of the amino acid sequence of SEQ ID NO: 4 or 6: 
SEQ ID NO: 4
GTNDAEDCCLSVTQKPIPGYIVRNFHYLLIKDGCRVPAVVFTTLRGRQLCA
PPDQPWVERIIQRLQRTSAKMKRRSSX
 X is an amino acid residue that can be biotinylated, such as lysine, ornithine or diaminopropionic acid and optionally is biotinylated, optionally via a spacer molecule such as PEG 
SEQ ID NO: 6
GTNDAEDCCLSVTQKPIPGYIVRNFHYLLIKDGCRVPAVVFTTLRGRQLCA
PPDQPWVERIIQRLQRTSAKMKRRSSX
 X is K(Biotin)

In the context of the various embodiments of the present invention the term "antibody" includes all immunoglobulins or immunoglobulin-like molecules with specific binding affinity for the relevant receptor, including chemokine receptor (including by way of example, IgA, IgD, IgE, IgG and IgM, combinations thereof, and similar molecules produced during an immune response in any vertebrate, for example, in mammals such as humans, goats, rabbits and mice).. Specific immunoglobulins useful in the various embodiments of the invention include IgG isotypes. The antibodies useful in the various embodiments of the invention may be monoclonal or polyclonal in origin, but are typically monoclonal antibodies. Antibodies may be human antibodies, non-human antibodies, or humanized versions of non-human antibodies or chimeric antibodies. Various techniques for antibody humanization are well established and any suitable technique may be employed. The term "antibody" also refers to a polypeptide ligand comprising at least a light chain or heavy chain immunoglobulin variable region which specifically recognizes and binds an epitope of an antigen, and it extends to all antibody derivatives and fragments that retain the ability to specifically bind to the relevant receptor, including chemokine receptor. These derivative and fragments may include Fab fragments, F(ab')₂ fragments, Fv fragments, single chain antibodies, single domain antibodies, Fc fragments etc. The term antibody encompasses antibodies comprised of both heavy and light chains, but also heavy chain (only) antibodies. In specific embodiments, the antibodies may be engineered so as to be specific for more than one receptor, including chemokine receptor, for example bi-specific to permit binding to two different receptors, including chemokine receptors. Suitable commercially available antibodies which bind to the chemokine receptors of interest are listed in table 1 below. They may or may not be labelled. General reference may be made to " Antibodies a laboratory manual: By E Harlow and D Lane. pp 726. Cold Spring Harbor Laboratory. 1988".

**Table 1. Commercially available fluorophore labelled antibodies against specific chemokine receptors**

| **Antibody** | **Fluorophore** | **Supplier** |
|---|---|---|
| CCR9 | APC | R&D Systems |
| CD14 | FITC | Beckman Coulter |
| CCR7 | PerCP Cy5.5 | Biolegend |
| HLA-DR | APC Cy7 | Biolegend |

The chemokine receptor expressing cells may thus be targeted using alternative binding agents, such as antibodies, as defined herein, rather than the natural chemokine binding partner. This approach is a new approach to treating inflammatory conditions, such as IBD, in particular UC or CD and IBS.

Thus, in certain parts the disclosure provides an apheresis column loaded with a solid support comprising a binding reagent capable of specifically binding to a chemokine receptor immobilized directly or indirectly on the support to permit removal of a cell expressing the chemokine receptor from the peripheral blood of a patient, wherein the binding reagent is not a chemokine. The binding reagent capable of specifically binding to the chemokine receptor may be an agonist or an antagonist of the chemokine receptor. In specific embodiments, the binding reagent capable of specifically binding to the chemokine receptor is selected from an antibody.

Similarly, other parts of the disclosure provides a binding reagent capable of specifically binding to a chemokine receptor for use in the treatment of IBD, wherein the binding reagent is immobilized on a solid support contained within an apheresis column as defined above (an apheresis column loaded with a solid support comprising a binding reagent capable of specifically binding to a chemokine receptor immobilized directly or indirectly on the support to permit removal of a cell expressing the chemokine receptor from the peripheral blood of a patient/subject, wherein the binding reagent is not a chemokine), to which is applied peripheral blood from a patient thus removing chemokine receptor expressing cells from the peripheral blood of the patient.

Solid support materials for immobilizing the binding reagents of the various embodiments of the invention are known in the art. "Solid support" refers to, for example, materials having a rigid or semi-rigid surface or surfaces, and may take the form of beads, resins, gels, microspheres, or other geometric configurations. A useful support material is one that does not activate blood cells so as to make them coagulate or adhere to the support as peripheral whole blood is applied to the device. In certain embodiments, a support treated with an agent to provide it with anti-coagulation properties, in particular a heparinized support is employed. Alternatively, the blood of the patient may be treated with an anti-coagulant such as heparin prior to application to the support. Useful support materials comprise high molecular weight carbohydrates, in particular carbohydrates having a molecular weight of 100 kDa or more, such as agarose, in particulate form, optionally cross-linked, and cellulose. Other preferred support materials are polymers, such as carboxylated polystyrene, and glass. The support of the various embodiments of the invention may be provided in the form of particles or fibres. The support particles may have regular form, such as spheres or beads, or irregular form. They may be porous or non-porous. A preferred average particle size of the support is from 50 µm to 2 mm. In certain embodiments Sepharose™, a cross linked, beaded-form of agarose, is used as column matrix. It is chosen for its optimal distribution capacity and can provide a large available area for affinity binding. Solid supports may be provided in the form of magnetic beads, with the specific binding reagent immobilized on the magnetic beads. Following capture of the (CD14, HLA-DR, CCR9 and/or CCR7) receptor, including chemokine receptor, expressing cells, in particular CD14+HLA-DRhi monocytes from the blood, the beads can be removed from the blood with the aid of an appropriate magnetic separator.

Methods for immobilizing binding reagents on a solid support are known in the art. A binding reagent, being an antibody, can be immobilized on the support in a direct or indirect manner. Immobilization can be by means of a suitable linker in some embodiments. A preferred method of indirect immobilization of a binding reagent, such as a chemokine, relies upon the interaction between biotin and avidin molecules. "Avidin" or "avidin molecule" refers to any type of protein that specifically binds biotin to the substantial exclusion of other (small) molecules that might be present in a biological sample. Examples of avidin include avidins that are naturally present in egg white, oilseed protein (e.g., soybean meal), and grain (e.g., corn/maize), and streptavidin, which is a protein of bacterial origin. Thus, biotinylation of the binding reagent and use of an avidin molecule such as streptavidin immobilized on the solid support allows reliable attachment of the binding reagent to the solid support according to methods known in the art. Specifically, such a method may comprise providing the binding reagent in biotinylated form, providing a solid support having streptavidin immobilized on its surface, contacting the support with an aqueous solution of the biotinylated binding reagent, and rinsing the support with an aqueous solvent. In addition, binding pair interactions, such as antibody - antigen interactions, may also be utilised for indirect immobilisation of binding reagent onto a support. In such embodiments the support may be derivatised with one member of a binding pair, such as an antibody, as defined herein, which has known affinity for a particular peptide sequence or small molecule hapten. Incorporating the other member of the binding pair, such as an antigen, peptide sequence or the hapten onto or into the binding reagent facilitates immobilisation onto a solid support coated with the corresponding antibody or fragment or derivative thereof. Thus, the binding reagent may be modified to include the peptide sequence or hapten into the linear molecule or may be added as a side chain or label.

Alternatively the antibodies, can be immobilised directly onto a solid support using bioconjugation techniques established in the field. For example direct immobilisation of proteins onto cyanogen bromide activated solid supports via amino functionalities within the primary sequence of the protein. Alternatively, sulphydryl functionalities within proteins can be used to directly immobilise the protein to alkyl halide derivatised supports or supports containing free thiol functionalities. In further embodiments, proteins containing α-thioester functionalities can be directly immobilised on supports containing 1,2 amino thiol moieties (eg N-terminal cysteine) using the native chemical ligation reaction. Alternatively proteins modified with ketones and aldehydes can be immobilised on solid supports derivatised with hydrazinyl, hydrazide and aminoxy functionalities using hydrazone / oxime bond forming ligation reactions (and vice versa). Alternatively 'Click' chemistry can be used to immobilise proteins onto solid supports, whereby the protein and the support are derivatised with the appropriate mutually reactive chemical functionalities (azides and alkynes). In other embodiments Staudinger ligation chemistry can be used to immobilise appropriately derivatised proteins onto the appropriately derivatised solid supports.

The solid support is contained within or carried by the apheresis column. Thus, by "loaded" is meant that the column carries or contains the solid support in a manner such that (peripheral) blood can flow through the column in contact with the solid support. Thus, the solid support provides a matrix within the column through which blood flows, in continuous fashion in certain embodiments. This permits cells expressing the specific receptor, i.e. the CCR9 receptor, to be removed from the blood passing through the column, such that blood exiting the column is depleted of the specific receptor, including chemokine receptor,-expressing cells. In specific embodiments, the apheresis column is loaded with a support comprising streptavidin immobilized on the support and one or more biotinylated binding reagents, such as chemokines, bound to the streptavidin on the support. The solid support may be comprised of a high-molecular weight carbohydrate, optionally cross-linked, such as agarose.

As discussed above, the binding reagent is coupled to the solid support. The relative amounts of binding reagent may be controlled to ensure that coupling between the solid support and the binding reagent will be immediate, minimising the risk of binding reagent decoupling from the solid support. Thus, it may be ensured that there is a relative excess of immobilization sites for the binding reagent on the solid support. Alternatively, or additionally, following immobilization of the binding reagent on the solid support, the solid support may be washed to remove any unbound binding reagent.

The apheresis column utilised in the various embodiments of the present invention acts as a leukapheresis treatment for inflammatory bowel disease, including Crohn's disease (CD) and ulcerative cholitis (UC). The column acts to specifically remove CD14+HLA-DRhi monocytes by exploiting the interaction between CCR9 expressed on the cell surface and a specific binding reagent immobilized on a solid support contained within or carried by the column. Similarly, the apheresis column utilised in the various embodiments of the present invention acts as a leukapheresis treatment for IBS. The column acts to specifically remove CCR9-expressing monocytes, in particular CD14+HLA-DRhi monocytes by exploiting the interaction between CCR9 expressed on the cell surface and a specific binding reagent immobilized on a solid support contained within or carried by the column.

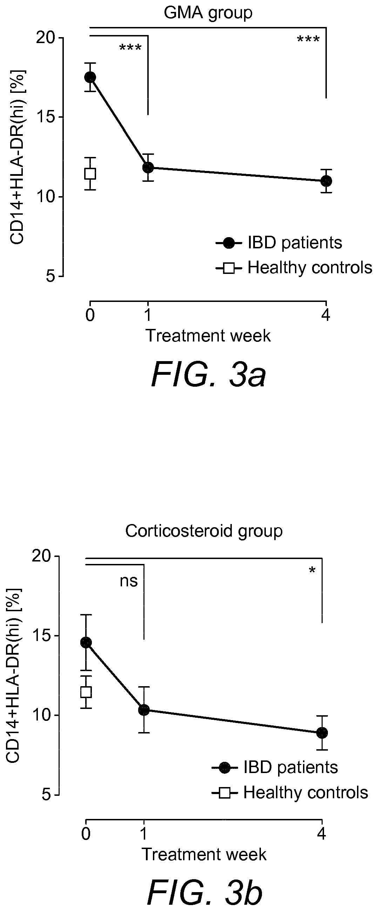

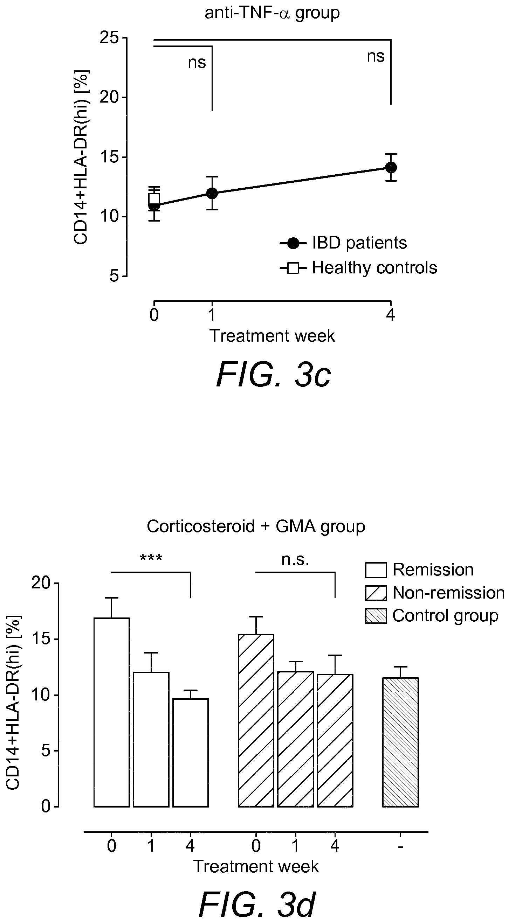

The overall column typically comprises, consists of, or consists essentially of three combined components; 1) a housing which contains or carries 2) the solid support and 3) one or more binding reagents (immobilized thereon) which specifically bind one or more chemokine receptors. The housing may be manufactured from any suitable material for clinical use. The housing is composed of a plastic material. The housing includes an in flow site for entry of blood and an out flow site for blood (depleted of target cells) to exit the column. The housing may be designed to maintain a continuous blood flow through the solid support matrix. The housing (as shown for example in Figure 3) may include a top portion which comprises a distribution plate (2) at the inflow site (1) to spread the blood evenly over the entire matrix area. The distribution plate may act as a first safety barrier preventing larger particles flowing through the column and into the patient. However, the distribution plate is not essential and may be removed in some embodiments to decrease the overall resistance in the system. The column may contain one or more safety filter units (3 and 4) placed at the inflow (1) and/or outflow (5) sites of the plastic housing. Such filter units may act to prevent particles larger than blood cells passing in and/or out of the column. The safety filter units may contain a plurality of filters, such as two, three or four filters designed to be a robust barrier and stop all particles larger than blood cells passing through the column. Inclusion of safety filters (3 and 4) at both ends of the column serves to minimize the risk of leakage of particles into the patient, including in the event that the device is incorrectly connected resulting in blood flow in the opposite direction to that intended. The safety filters may comprise of any suitable pore size to prevent particles larger than blood cells from passing through the column, as would be readily understood by one skilled in the art. Suitable filters are commercially available. In specific embodiments, the pore size of the filter(s) is between approximately 60µm and 100µm, more specifically approximately 80 µm. The solid support and binding reagent components are discussed in further detail herein.

The volume of the housing may be varied depending upon the blood volumes intended to pass through the column. Typically, the volume of the housing is between approximately 40ml and 200ml, more specifically 50ml to 150ml or 60ml to 120ml.

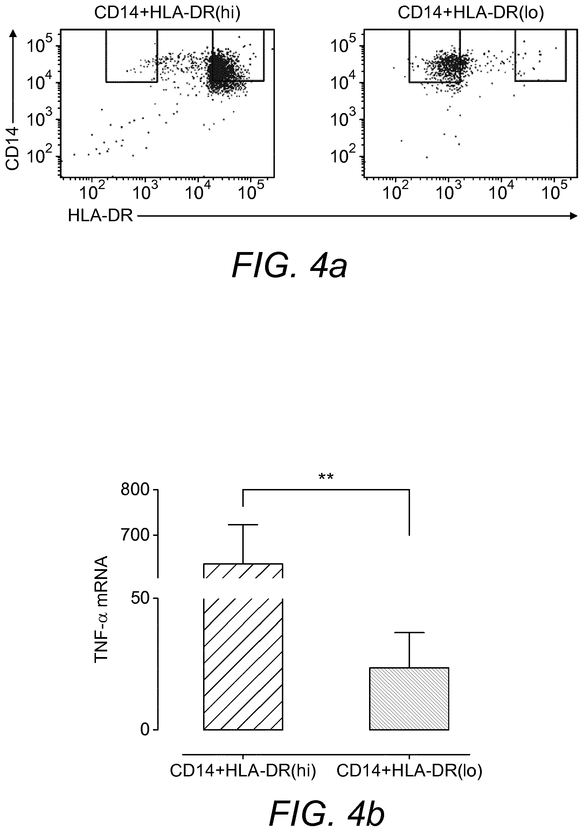

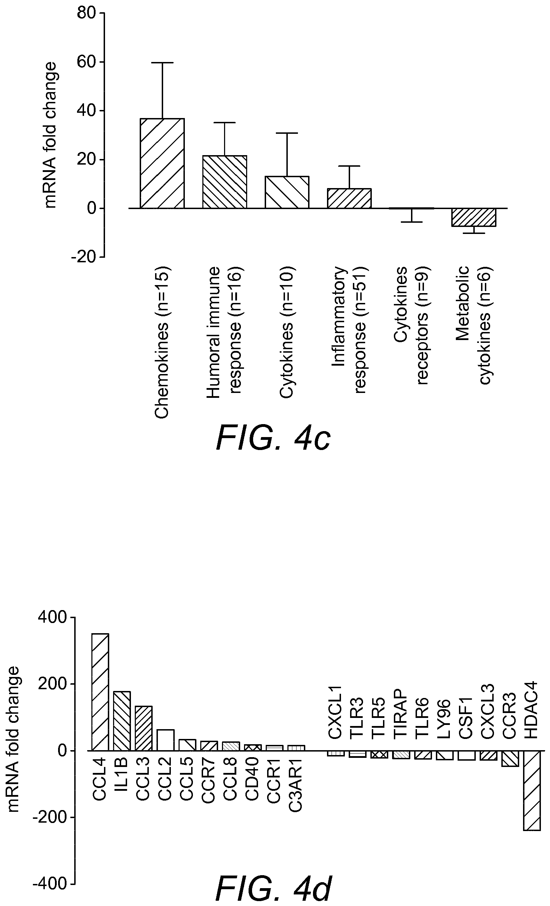

The column is generally applied in the form of an apheresis circuit. In this context, the overall system includes the apheresis column, tubing and an appropriate pump to pump the blood around the circuit. The system is illustrated in figure 4. The patient (1) is connected to the extracorporeal circuit via sterile needles to veins in the right and the left arms. A saline bag (3) is also connected and the saline solution is pumped with a suitable pump (2). Blood is drawn from one arm of the patient through the sterile tubing system by the blood pump (4) and passed through the column (6) and back to the patient. The tubing system may be connected to the column via any suitable coupling, such as standard dialysis luer-lock couplings. The couplings on the column may be colour-coded for correct assembly. For example, red tubing for inflow to the red column top and blue tubing for outflow back to the patient. An air detector (8) may be present in the circuit. Inlet pressure (5) and/or Pven sensors (7) may additionally be employed to monitor the pressure in the circuit.

An apheresis pump, such as the 4008 ADS pump manufactured by Fresenius Medical Care or the Adamonitor pump, may monitor the patient's inflow and outflow. The pump may also monitor the pressure in the extracorporeal circulation. The pump may be able to discriminate air by a bubble catcher and air detector. A clot catcher filter may be positioned inside the bubble catcher. The pump may also incorporate an optical detector to distinguish between light, e.g. saline solution or air present in the tubing system and dark e.g. blood present in the tubing system.

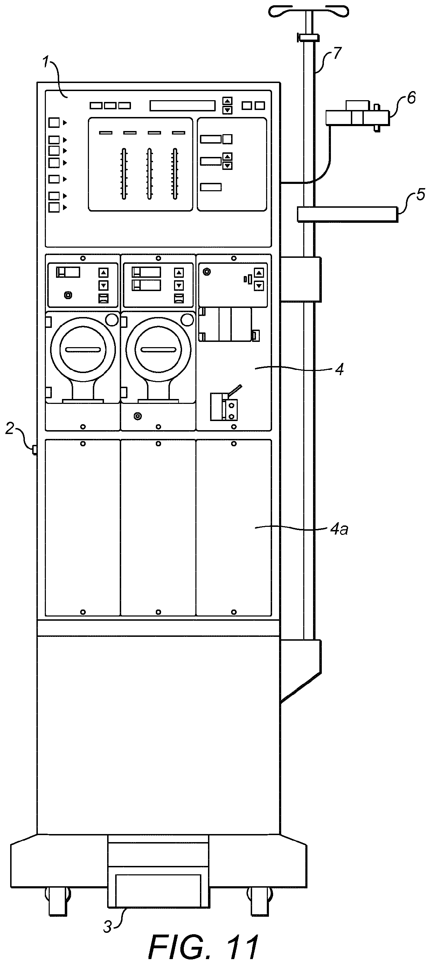

A schematic diagram of a suitable pump, showing the air detector and optical filter is shown in figure 11. If the pump system detects air bubbles and optical fluctuations or if extracorporeal pressure values are out of the set range, then the pump may stop immediately. Alternatively or additionally a visual/ audible alarm may be emitted.

Thus, (in this context) in certain embodiments the invention also provides a method of diagnosing, monitoring progression of, or monitoring treatment of inflammatory bowel disease comprising determining the levels of monocytes expressing CCR9 in a sample obtained from a subject, wherein high levels of monocytes expressing CCR9, or increased levels of monocytes expressing CCR9 compared to the levels of monocytes expressing CCR9 in a healthy subject, indicate the presence or progression of inflammatory bowel disease, and wherein the subject has been treated using a binding agent according to the invention.

"Diagnosing" is defined herein to include screening for a disease/condition or pre-indication of a disease/condition, identifying a disease/condition or pre-indication of a disease/condition and checking for recurrence of disease/condition following treatment. The methods of the various embodiments of the invention may also have prognostic value, and this is included within the definition of the term "diagnosis". The prognostic value of the methods of the various embodiments of the invention may be used as a marker of potential susceptibility to inflammatory bowel disease, including Crohn's disease (CD) and ulcerative cholitis (UC) by identifying levels of CCR9 expression or levels of CD14⁺HLA-DR^{hi} monocytes.

"Monitoring progression of" includes performing the methods to monitor the stage and/or the state and progression of the inflammatory bowel disease, including Crohn's disease (CD) and ulcerative cholitis (UC). Monitoring progression may involve performing the diagnostic methods multiple times on the same patient to determine whether the levels CD14⁺HLA-DR^{hi} monocytes or monocytes expressing CCR9 or CCR9 in the case of IBS are increasing, decreasing or remaining stable over a certain time period. This may be in the context of a treatment regime.

When monitoring progression of, or monitoring treatment of inflammatory bowel disease, including Crohn's disease (CD) and ulcerative cholitis (UC), the control samples may be taken from the subject at an earlier time point. They may, however, be based upon known reference values as discussed herein. Thus, relative levels of CD14⁺HLA-DR^{hi} monocytes or monocytes expressing CCR9, relative levels of HLA-DR^{hi}, CCR9^{hi} cells may be with reference to samples taken from the same subject at a different point in time. In certain embodiments, decreased levels of CCR9, decreased levels of CD14⁺HLA-DR^{hi} monocytes or monocytes expressing CCR9, decreased relative levels of CCR9 expression including decreased relative levels of average CCR9 expression per cell, or decreased relative levels of CD14^{hi}, HLA-DR^{hi}, CCR9^{hi} cells correlate with successful treatment.

When monitoring progression of inflammatory bowel disease, including Crohn's disease (CD) and ulcerative cholitis (UC), increased levels of CD14⁺HLA-DR^{hi} monocytes or monocytes expressing CCR9, CCR9 expression including increased relative levels of HLA-DR^{hi}, CCR9^{hi} cells may indicate the progression of condition or disease. Thus, if levels of CD14⁺HLA-DR^{hi} monocytes or monocytes expressing CCR9 are increased in a sample taken later than a sample from the same patient this may indicate progression of the condition.

Thus, in specific embodiments, high or higher levels of CCR9 expressing cells, high or higher levels of CD14⁺HLA-DR^{hi} monocytes or monocytes expressing CCR9 or high or higher levels of CCR9 expression, for example average CCR9 expression per cell, or high or higher levels of CCR9^{hi} cells correlate with active disease or more active disease. Similarly, lower or low levels of CCR9 expressing cells, low or lower levels of CD14⁺HLA-DR^{hi} monocytes or monocytes expressing CCR9 or low or lower levels of CCR9 expression, for example average CCR9 expression per cell, or low or lower levels of CCR9^{hi} cells may correlate with a lack of active inflammation or disease. This may be defined as "less active disease". It can readily be envisaged that control samples may be assessed and levels of CCR9 expressing cells, levels of CD14⁺HLA-DR^{hi} monocytes or monocytes expressing CCR9, levels of expression of CCR9 and/or levels of cells with high expression of CCR9 (defined as CCR9^{hi}) determined across the range of severities of IBS. This may assist in correlating the levels of CCR9 expressing cells, levels of CD14⁺HLA-DR^{hi} monocytes or monocytes expressing CCR9, levels of expression of CCR9 and/or levels of cells with high expression of CCR9 (defined as CCR9^{hi}) in the test sample with the relative severity of the condition.

The inventors have shown that levels of CD14⁺HLA-DR^{hi} monocytes or monocytes expressing CCR9 may be linked to improved success with certain treatments for inflammatory bowel disease.

The various embodiments of the invention will now be described in more detail by reference to the following embodiments and examples:

### DESCRIPTION OF THE FIGURES

**Figure 1****.** Representative flow cytometry plots showing the gating strategies used throughout the study for the CD14+HLA-DRhi (lower left), CD14loCD16+ (lower middle) and CD14+CD16- (lower right) monocyte populations.
**Figure 2****. CD14+HLA-DRhi monocytes are increased and correlate to disease activity in patients with ulcerative colitis and Crohn's disease.**
   (A) Frequency of CD14+HLA-DRhi monocytes in peripheral blood of IBD patients compared to controls, as determined with flow cytometry. Bars represent mean values ± SEM from controls (n=11) and patients (n=31) with active ulcerative colitis (n=20; UC-DAI 6-12) or Crohn's disease (n=11; HBI 8-16) (p=0.006).
   (B) Regression analysis of CD14+HLA-DRhi monocytes and clinical disease activity in patients with ulcerative colitis (p=0.024; r2=0.072). Data represents measurements (n=84) from 28 unique patients at different time points during treatment.
   (C) Regression analysis of CD14+HLA-DRhi monocytes and clinical disease activity in patients with Crohn's disease (p=0.016; r2=0.190). Data represents measurements (n=29) from 11 unique patients at different time points during treatment
**Figure 3****. CD14+HLA-DRhi monocytes are targets for therapy in IBD.**
   CD14+HLA-DRhi monocyte levels in IBD patients during treatment with (A) GMA apheresis (n=18), (B) corticosteroids (n=16) or (C) anti-TNF-a biological therapy (n=14). Control patient reference levels (n=11) are included in all graphs. (D) IBD patients receiving GMA or corticosteroid therapy, divided into remission (n=12) and non-remission (n=7) patients as well as healthy controls (n=11). Error bars represent group mean values ± SEM.
**Figure 4****. CD14+HLA-DRhi monocytes produce high levels of inflammatory mediators.**
   (A) Representative flow cytometry plots depicting CD14+HLA-DRhi and CD14+HLADR lo purity after flow cytometry sorting.
   (B) PCR analysis of TNF-α in CD14+HLA-DRhi monocytes after LPS activation for 2 hrs (n=4, p=0.0047).
   (C) Functional grouping of target transcripts from PCR array analysis of CD14+HLADR hi and CD14+HLA-DRlo monocytes from three independent healthy donors after LPS activation for 6 hrs.
   (D) The 20 target transcripts that represented the strongest up- and down-regulation in PCR array analyses of CD14+HLA-DRhi monocytes as compared to the CD14+HLADR lo population after LPS activation for 6 hrs. Fold changes range between 347.3-10.9 and -10.3--232.3, respectively.

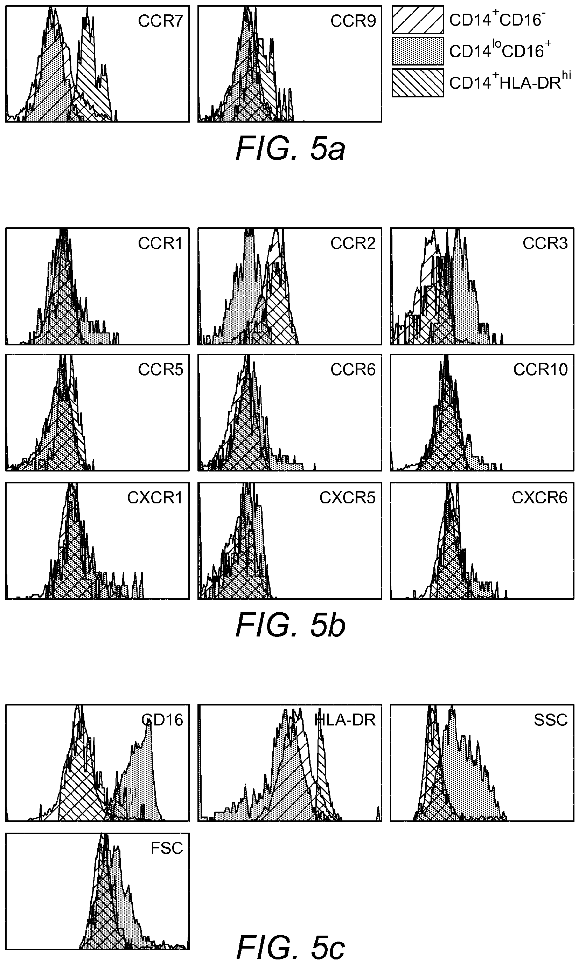

**Figure 5****. The relative chemokine receptor expression in CD14+HLA-DRhi monocytes.**
   (A) The CD14+HLA-DRhi subset is distinguished from CD14loCD16+ and CD14+CD16-monocytes by their expression of CCR7 and CCR9. (B) Chemokine receptors whose expression levels do not differentiate the CD14+HLA-DRhi population. (C) Control staining showing the difference in expression of CD16 and HLA-DR as well as morphology defined by side-scatter (SSC) and forward-scatter (FSC) appearance between the monocyte populations discussed in this study. Data presented are from one representative IBD patient.

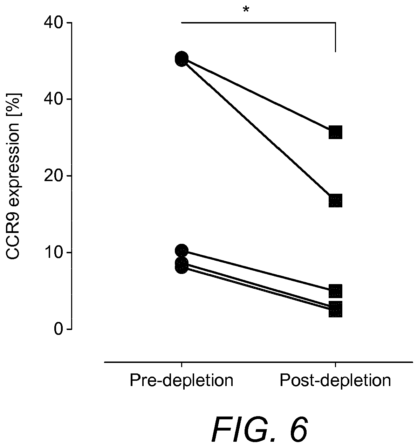

**Figure 6****. CCR9-CCL25 functional interaction assay in CD14+HLA-DRhi monocytes** Depletion of CCR9-expressing CD14+HLA-DRhi monocytes from IBD patients using CCL25-coated microbeads (n=5; p=0.0112).

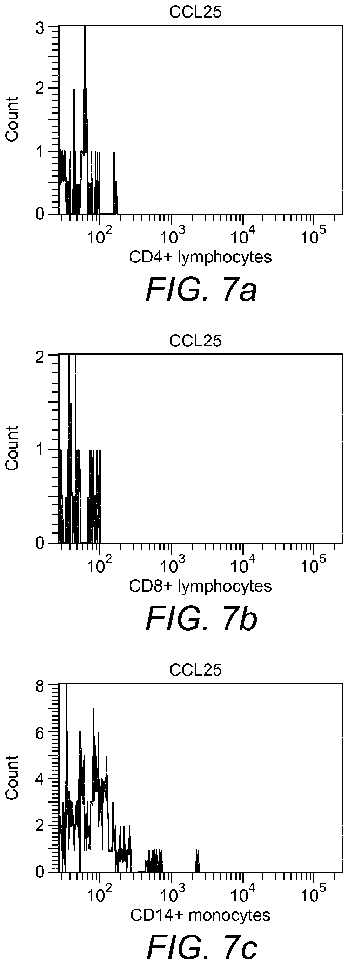

**FIG. 7a, 7b & 7c** - the binding of biotinylated CCL25 by CD4+, CD8+ T-cells and CD14+ monocytes respectively, obtained from peripheral blood of a healthy donor;

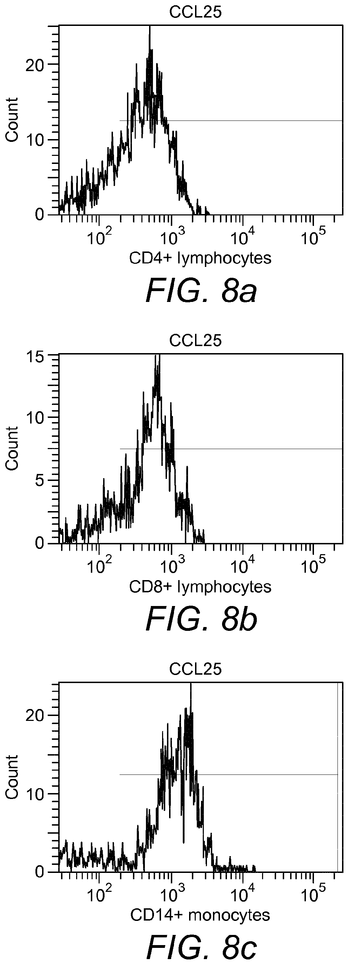

**FIG. 8a, 8b & 8c** **-** the binding of biotinylized CCL25 by CD4+, CD8+ T-cells and CD14+ monocytes respectively, obtained from peripheral blood of a patient with CD;

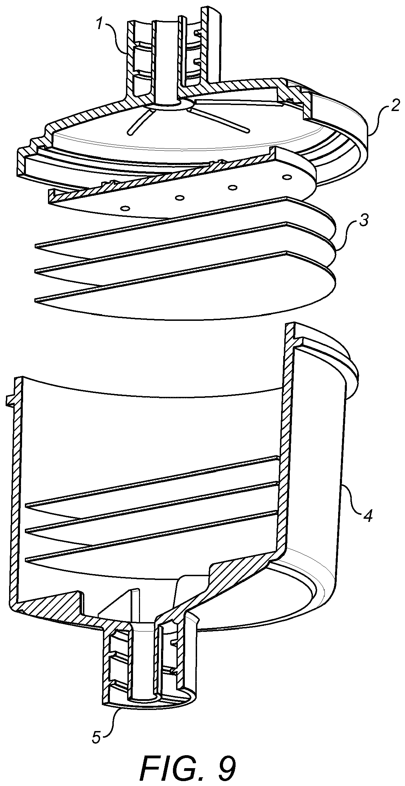

**FIG. 9** - The plastic house and top showing the distribution plate (2) and safety filter units (3 and 4).

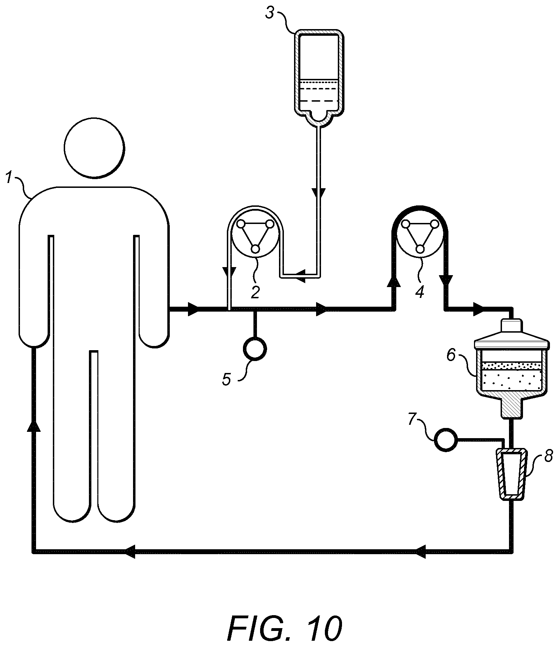

**FIG. 10** - The overall leukapheresis system.
**FIG. 11** - The pump with air detector and optical detector (4).

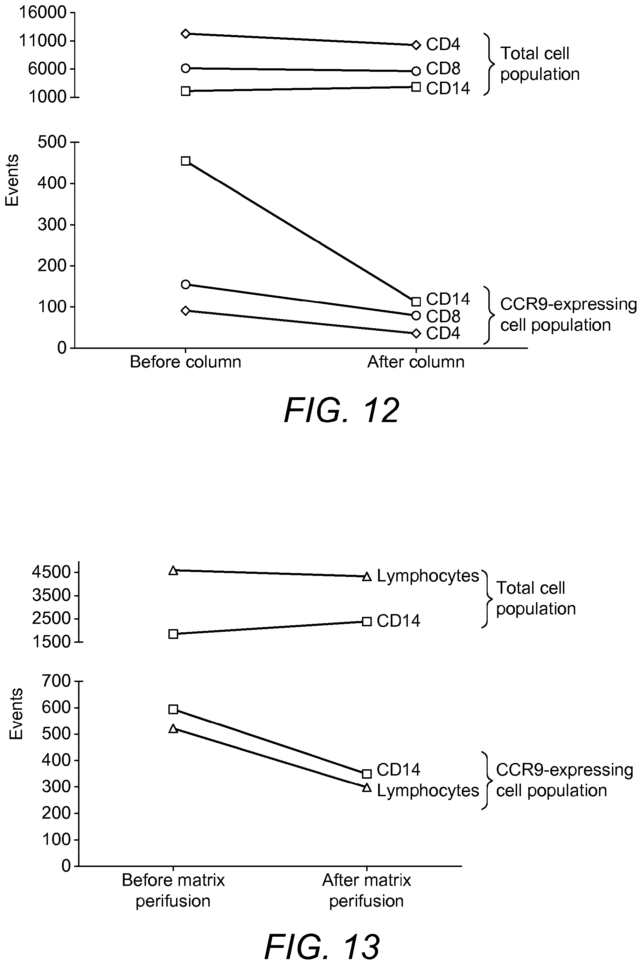

**FIG. 12** -Depletion of CCR9-expressing cell populations in one blood donor. Total cell populations are unaffected after the column passage.
**FIG. 13** - Depletion of CCR9-expressing cell populations in one IBD patient. Total cell populations are unaffected after the column passage.

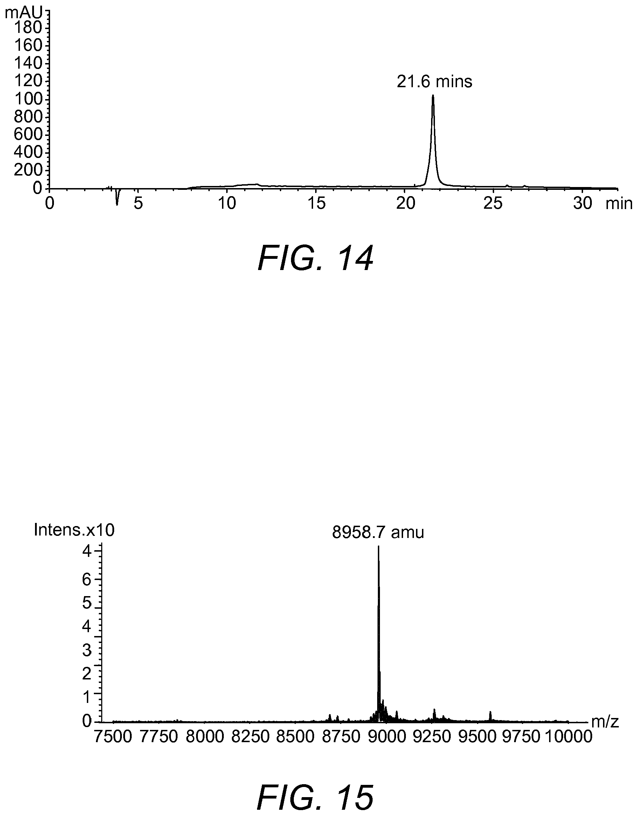

**FIG. 14** - HPLC of purified folded Biotin-TECK(Nleu).
**FIG. 15** - Electrospray ionisation with tandem mass spectrometry (ES/MS) data of purified folded Biotin-TECK(Nleu).
**FIG. 16** - example of gating criteria for CCR2 expressing monocytes

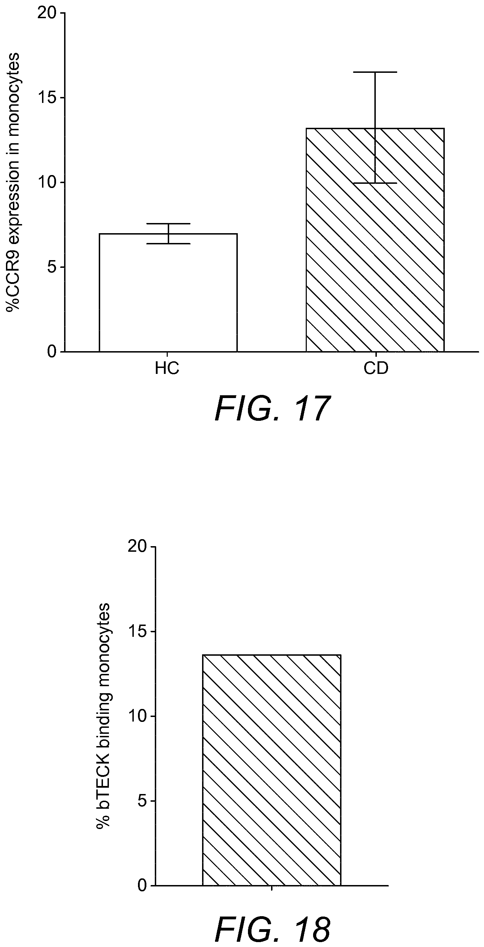

**FIG. 17** - Expression of CCR9 expressing monocytes in seven patients with Crohn's disease (CD) and in 20 healthy controls (HC). Blood from patients with CD and healthy controls was analysed for the expression of various chemokine receptors by flow cytometry. The monocytes were characterized as CD14 positive cells.
**FIG. 18** - Binding of the chemokine bTECK (CCL25) to monocytes from a patient with Crohn's disease. Blood from a patient with CD was incubated with bTECK and analysed with flow cytometry. The monocytes were characterized as CD14 positive cells.

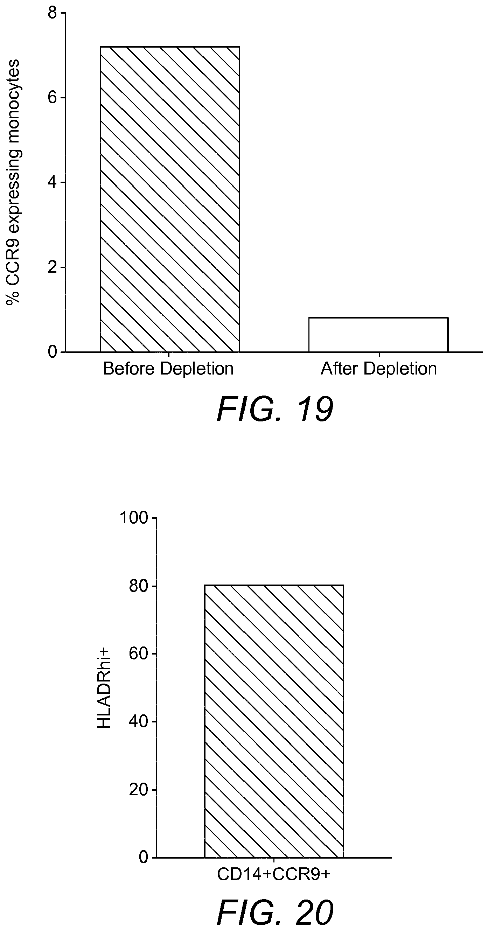

**FIG. 19** - Depletion of CCR9 expressing monocytes with Sepharose Streptavidin-matrix conjugated with bTECK. Blood cells from a patient with CD were incubated with bTECK-Sepharose Streptavidin-matrix. Unbound cells were retrieved by washing the matrix with Phosphate Buffer Saline. The cells (After Depletion) were then analysed with flow cytometry and compared with cells that had not been incubated with bTECK-matrix (Before Depletion).
**FIG. 20** - The CCR9 expressing monocytes show a 80% expression of HLADRhi. The monocytes were characterized as CD14 positive cells.

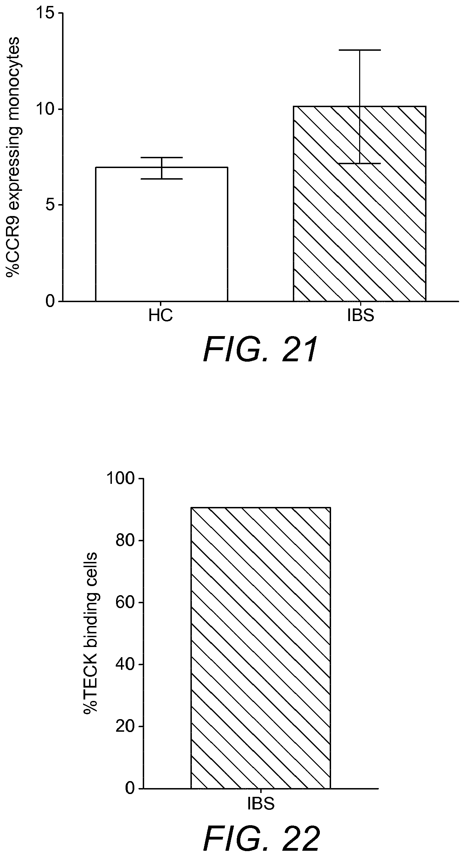

**FIG. 21** - Expression of CCR9 expressing monocytes in 2 patients with Irritable bowel syndrome (IBS) and in 20 healthy controls (HC). Blood from patients with IBS and healthy controls was analysed for the expression of various chemokine receptors by flow cytometry. The monocytes were characterized as CD14 positive cells.
**FIG. 22** - Binding of the chemokine bTECK (CCL25) to monocytes from a patient with IBS. Blood from a patient with IBS was incubated with bTECK and analysed with flow cytometry. The monocytes were characterized as CD14 positive cells.

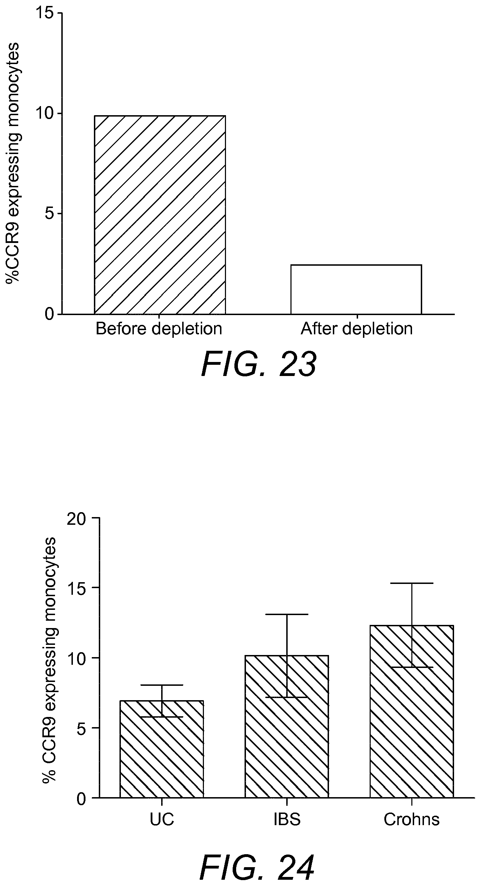

**FIG. 23** - Depletion of CCR9 expressing monocytes with Sepharose Streptavidin-matrix conjugated with bTECK. Blood cells from a patient with IBS were incubated with bTECK-Sepharose Streptavidin-matrix. Unbound cells were retrieved by washing the matrix with Phosphate Buffer Saline. The cells (After Depletion) were then analysed with flow cytometry and compared with cells that had not been incubated with bTECK-matrix (Before Depletion).
**FIG. 24** - Expression of CCR9 expressing monocytes in 4 patients with Ulcerative Colitis (UC), 2 patients with Irritable bowel syndrome (IBS) and 8 patients with Crohns Disease (Crohns). Blood from patients was analysed for the expression of various chemokine receptors by flow cytometry. The monocytes were characterized as CD14 positive cells.

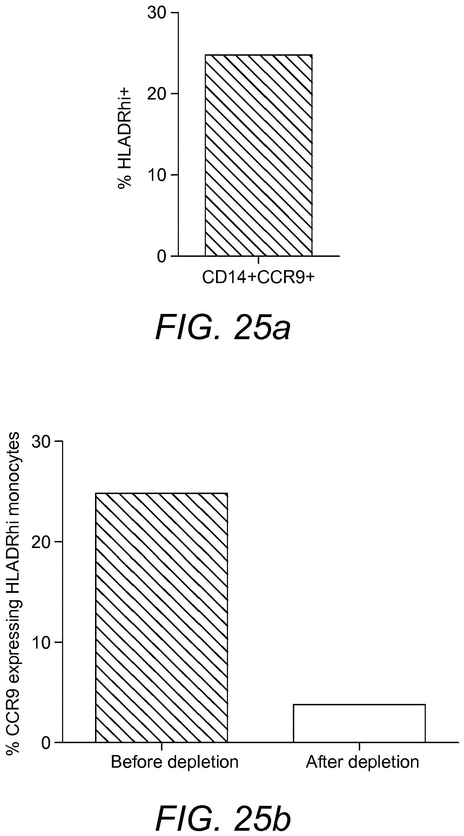

**FIG. 25a** - The CCR9 expressing monocytes show a 25% expression of HLADRhi. **FIG. 25b** - The CCR9 expressing monocytes which also have a high expression of HLADR can be depleted with bTECK conjugated sepharose matrix. The monocytes were characterized as CD14 positive cells.

### DESCRIPTION OF PREFERRED EMBODIMENTS

### EXAMPLE 1 CCR9-expressing CD14+HLA-DRhi blood monocytes promote intestinal inflammation in IBD

### Introduction

Crohn's disease (CD) and ulcerative colitis (UC) (Inflammatory bowel diseases; IBD) are chronic, inflammatory disorders of the gastrointestinal tract resulting from a disrupted balance between the mucosal immune system and commensal flora. To date, the immunological pathophysiology behind IBD remains poorly understood. Traditionally, adaptive immunity was believed to play an important role in the onset of IBD. Studies in patients and animal models have shown that CD is driven by T helper 1-signaling with IL-12 and IFN-γ production, whereas UC is characterized by T helper 2-responses and IL-13.(1) However, the Th1/Th2 paradigm has been questioned over the last decade.(2) Since the discovery of the *NOD2*/*CARD15* susceptibility locus that encodes a pattern recognition receptor mainly expressed on dendritic cells and monocytes, the focus of IBD research has shifted towards innate immunity.(3, 4) Currently, innate mechanisms are believed to be responsible for the onset of acute mucosal inflammation in genetically susceptible individuals, whereas the chronic state might be maintained by adaptive elements.(5)

Monocytes are bone marrow-derived leukocytes of the myeloid lineage that migrate to the tissue and differentiate into macrophages or dendritic cells (DCs). Increased turnover rates and elevated levels of circulating monocytes have been demonstrated in IBD.(6, 7). Furthermore, monocytes have the ability to migrate to the inflamed mucosa and mediate inflammation, but the phenotype of these monocytes as well as the mechanisms underlying this relocation remains to be elucidated.(8-10) Currently, two main human monocyte subpopulations have been characterized. The CD14+CD16- cells have been shown to produce the regulatory cytokine IL-10 and are most commonly referred to as classical monocytes. The CD14loCD16+ subset is characterized by production of pro-inflammatory cytokines as well as high surface expression of inflammatory markers such as CD43.(11-13) However, a larger degree of heterogeneity among human monocyte populations with regards to their surface antigen expression has lately been observed.(14) Monocyte HLA-DR expression has been demonstrated to play an important role in conditions characterized by immune responses against bacterial agents.(15, 16) Although the CD14+CD16- subset has been reported to express HLA-DR, the specific contribution of CD14+HLA-DRhi monocytes to intestinal inflammation has not been studied. Since it is well established that induction of colitis in human as well as in animal models requires the presence of bacteria, we set out to study CD14+HLA-DRhi monocytes in patients with chronic intestinal inflammation.(17)

### Materials and Methods

### Patients

In total, 51 IBD patients (UC=31; CD=20) were included in this study (Table 1). The patients were monitored during treatment with corticosteroids (n=16), the anti-TNF-α antibodies infliximab or adalimumab (Remicade® or Humira®; n=17), or Granulocyte/Monocyte apheresis (GMA; Adacolumn®; n=18). Four to six biopsies from affected rectum and sigmoideum were collected together with blood samples before the start of treatment, followed by weekly sampling for four consecutive weeks. Patients were assessed using the UC-DAI (UC) and Harvey-Bradshaw (HBI; CD) indices. Clinical remission was reviewed at week 11 post-treatment and defined as <3 for UC-DAI and <5 for HBI.(8, 41) Fourteen controls without IBD were included in the study. All patients were enrolled through formal consent and the study was approved by the regional ethics committee.

### Leukocyte isolation and activation

For flow cytometry studies, peripheral blood mononuclear cells (PBMC) were obtained from heparinised whole blood by incubation in hypotonic buffer (160 mM NH4CI, 10 mM Tris-HCI, pH=7.4). For PCR and CCL25 depletion experiments, PBMC were obtained from anticoagulated healthy donor buffy coats by density gradient centrifugation using Ficoll-Paque (GE Healthcare). For PCR experiments, CD14+ monocytes were negatively isolated using Monocyte Isolation Kit II (Miltenyi Biotec). Monocytes were subsequently activated with LPS (lipopolysaccharide; Sigma) (200 ng/mL/106 cells) for 2 hrs (TNF-α PCR) or 6 hrs (PCR array) in RPMI medium (Hyclone) supplemented with 1% L-glutamine and 1% PEST (penicillin-streptomycin).

### Flow cytometry

PBMC were stained for flow cytometry analysis or sorting using combinations of the antibody conjugates described in Table 2. All stainings were carried out according to the instructions of the manufacturer for the respective antibody conjugate. An IgG2a-FITC (BD Biosciences) isotype control was used to define CCR9 positivity. Flow cytometry analyses and sorting experiments were carried out using a FACSAria cytometer and data was analysed using FACSDiva software (BD Biosciences).

### PCR experiments

For TNF-α PCR experiments, RNA isolation was performed using TRIZOL reagent (Invitrogen). For the CCL25 experiment, intestinal biopsies were collected through flexible sigmoidoscopy from UC patients and immediately submerged in RNAlater (Ambion). RNA was subsequently isolated using RNeasy Mini Kit (Qiagen) according to the manufacturer's protocol. For the TNF-α and CCL25 experiments, 100 ng RNA per sample was included in a reverse transcriptase reaction using iScript cDNA synthesis kit (Bio-Rad). For PCR array analyses, RNA was isolated from sorted CD14+HLA-DRhi and CD14+HLA-DRlo populations using RNeasy Mini Kit (Qiagen). For each of the analyzed populations, equal amounts of RNA from three independent donors were pooled and cDNA was synthesized using RT2 First Strand Kit (SABiosciences) from 150 ng of RNA. Subsequently, cDNA was put into a RT2 qPCR Master Mix (SABiosciences) reaction and loaded onto a Human Inflammatory Response and Autoimmunity 96-well PCR array plate according to the instructions of the manufacturer (SABiosciences). In all PCR experiments, quantitative PCR was performed on an iCyclerIQ Optical System using 2X IQ SYBR Green supermix and iCycler IQ Optical System Software v3.1 (Bio-Rad) for data retrieval. In the TNF-α and CCL25 experiments, expression levels were normalized to RNA polymerase II using either the 2-_Ct (TNF-α; Figure 4B) or 2-_Ct (CCL25; Supplementary Figure 1B) methods. Primers used were TNF-α forward (5'-CTCTCTCCCCTGGAAAGGAC-3'); TNF-α reverse (5'-GCCAGAGGGCTGATTAGAGA-3'); CCL25 forward (5'- AAGGTTTTTGCAAAGCTCCA-3'); CCL25 reverse (5'-TACTGCTGCTGATGGGATTG- 3'); RPII forward (5'-GCACCACGTCCAATGACAT-3'); RPII reverse (5'- GTGCGGCTGCTTCCATAA-3'). For PCR array analyses, expression levels were normalized to the arithmetic mean expression of the B2M, HPRT1, RPL13A, GAPDH and ACTB housekeeping genes using the 2-_Ct method.

### CCL25 depletion assay

Biotinylated CCL25 (Almac Sciences) was bound to a solid support consisting of a streptavidin-sepharose matrix (GE Healthcare). PBMC from six healthy donors was perfused through the device and CCR9 expression was analysed before and after using flow cytometry.

### Statistical analyses

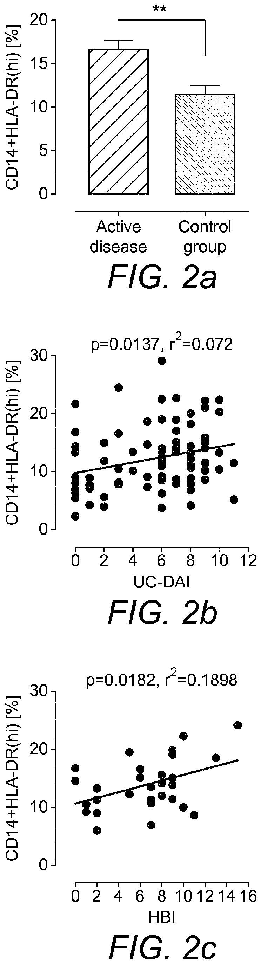

All group analyses were carried out using two-tailed dependent Student's t-test (Figures 2-4 and 6; Supplementary Figure 1) or two-tailed independent Student's t-test (Figure 3). Regression analyses were performed using ordinal regression test for non-parametric data (Figure 2B-C). All calculations were carried out in GraphPad Prism v5 software (GraphPad Software, Inc.). Values of p≤0.05 were regarded as significant and depicted as follows: p≤0.05 = *, p<0.01 = **, p≤0.001 = ***. In all figures, error bars represent ± SEM.

### Ethical considerations

The study was approved by the Stockholm Regional Ethical Review Board in Stockholm, Sweden (http://www.epn.se). The ethical approval applies to all centra from which patients were recruited (South Hospital, Stockholm, Sweden; Karolinska Hospital, Stockholm, Sweden; Danderyd Hospital, Stockholm, Sweden). All patients were enrolled through formal written consent.

### Results

### The frequency of CD14+HLA-DRhi monocytes correlates to clinical disease activity in ulcerative colitis and Crohn's disease

To investigate the role of CD14+HLA-DRhi monocytes in the IBD patients, we used flow cytometry to identify the population in peripheral blood (Figure 1). When analyzing blood from patients and controls we found that active inflammation in the colon correlated to a significantly higher frequency of these monocytes compared with the control group (Figure 2A, p=0,006).

In order to assess the correlation between monocyte levels and disease activity, we carried out regression analyses of CD14+HLA-DRhi frequency against two commonly used disease activity indices for the assessment of ulcerative colitis and Crohn's disease, respectively. We could observe a significant correlation with disease activity in both UC (UCDAI) (Figure 2B; p=0.0137, r2=0.072) and CD (Harvey-Bradshaw) (Figure 2C; p=0.0182, r2=0.1898). Thus, the frequency of circulating pro-inflammatory CD14+HLA-DRhi monocytes correlates with established disease activity indices of IBD.

### CD14+HLA-DRhi monocytes are potential therapeutic targets and markers of inflammation in colitis

Next, we investigated whether the CD14+HLA-DRhi population was affected by conventional IBD therapy. Patients with active intestinal inflammation who received either corticosteroids or anti-TNF-α antibodies (infliximab or adalimumab) were monitored for five consecutive weeks. A patient group treated with Granulocyte/Monocyte apheresis was included for comparison considering the selective removal of monocytes associated with Adacolumn®.(18) When plotting these treatment regimes separately, the patient group receiving GMA therapy accounted for the most prominent decrease (Figure 3A). The monocyte population was also attenuated already after one week of therapy among corticosteroid patients. The suppression was maintained, reaching levels well below those of healthy control patients at week 4 (Figure 3B, p<0.05). The decreased population of CD14+HLA-DRhi during treatment was not influenced by the diagnosis UC or CD, extension of the disease in the colon, concomitant azathioprine treatment or gender (data not shown). Interestingly, biological therapy with antibodies against TNF-α did not significantly affect the proportion of CD14+HLA-DRhi monocytes (Figure 3C). Among these patients, CD14+HLADRhi never reached the reference level observed in the controls.

Lastly, we investigated whether the CD14+HLA-DRhi population was selectively affected in patients achieving long-term remission at week 11 post-treatment. Among patients receiving corticosteroids or GMA apheresis, the monocyte population was significantly decreased in those who later achieved or maintained remission. This was not observed among the non-remission patients (Figure 3D).

### CD14+HLA-DRhi monocytes produce high levels of inflammatory mediators

With the purpose of investigating the capacity of CD14+HLA-DRhi monocytes to produce inflammatory mediators, monocytes from healthy blood donors were cultured in the presence of lipopolysaccharide (LPS). The HLA-DRhi population and its CD14+HLA-DRlo counterpart were subsequently sorted with flow cytometry (Figure 4A). The two cell populations were investigated with regards to the production of the proinflammatory cytokine TNF-α. Interestingly, the CD14+HLA-DRhi population produced 500-fold increased levels of TNF-α transcripts upon LPS-stimulation compared to the CD14+HLA-DRlo cells (Figure 4B). Furthermore, PCR array analyses were carried out on sorted CD14+HLA-DRhi monocytes from three independent donors after activation with LPS in order to establish the distinctive phenotype of the population. In accordance with our hypothesis several gene transcripts described as being involved in monocyte-mediated immune responses were up-regulated in the CD14+HLA-DRhi monocytes. Increased gene expression was mainly found among chemotactic cytokines and genes involved in the humoral immune response (Figure 4C; Supplementary table). The most prominent fold change difference between the CD14+HLADR hi and the CD14+HLA-DRlo monocytes was observed for the chemotactic cytokine CCL4. The transcript with the most apparent down-regulation among HLA-DRhi monocytes was the HDAC4 gene that encodes a histone deacetylase that functions as a transcriptional repressor.(19) Together, this data shows that CD14+HLA-DRhi monocytes have strong proinflammatory
potential.

### CD14+HLA-DRhi monocytes express the gut-homing chemokine receptor CCR9

Next, we studied the surface expression of various chemokine receptors on CD14+HLA-DRhi monocytes in relation to the CD14+CD16- and CD14loCD16+ subsets. Although we could observe significant overlap between many of these markers in terms of their expression in the respective subsets, the CD14+HLA-DRhi population was clearly distinguished by its expression of CCR7 and CCR9 (Figure 5A). CCR7 is mainly described as a lymph-node homing receptor for dendritic cells and T helper cells, and has previously not been reported to be expressed on circulating monocytes.(20) CCR9 has been shown to be important in lymphocyte homing to the gut through interactions with its ligand CCL25, expressed in the intestinal mucosa.(21, 22) Although CCR9-CCL25 interactions have been well characterized in T helper cells, their role in monocytes is unclear. The general CD14+ monocyte population exhibited significantly higher CCR9 expression compared to CD4+ T lymphocytes, which together with CD8+ T lymphocytes have been described as the main CCR9 expressing cell types (p<0.05; Supplementary Figure 1A).(23) When comparing CCR9 expression in CD14+HLA-DRhi with CD14loCD16+ and CD14+CD16- monocytes, the HLA-DRhi subset displayed significantly increased levels (Figure 5A). In contrast, the expression of CCR2, a chemokine receptor responsible for general monocyte migration, was not increased on the HLA-DRhi monocytes indicating that gut-homing phenotype constitutes a specific feature of the monocytes rather than reflecting general immunological activation (Figure 5B).(24) We also analyzed colonic mucosal tissue biopsies with real-time PCR and found that the CCR9 ligand, CCL25/TECK, was expressed in the colon (Supplementary Figure 1B). To establish a functional interaction between CCL25 and CCR9, we carried out depletion experiments by perfusing peripheral blood cells through a solid support containing CCL25-coated sepharose beads. The frequency of CCR9-positive CD14+HLA-DRhi monocytes was significantly reduced after encounter with the CCL25-coated sepharose beads, showing that CCR9 on CD14+HLA-DRhi monocytes could bind CCL25 and be removed from the blood (p<0.05; Figure 6). In conclusion, pro-inflammatory CD14+HLA-DRhi monocytes express high levels of the gut-homing chemokine receptor CCR9 which directs them to the site of mucosal inflammation.

### Discussion

In this study, we identify CCR9-expressing CD14+HLA-DRhi blood monocytes as an important player in intestinal inflammation. The expression of HLA-DR on monocytes is vital to the inflammatory response and has been shown to determine the efficacy of antigen presentation to T helper cells.(25, 26) Monocytes with high expression of HLA-DR have also been shown to infiltrate the joints of patients with rheumatoid arthritis, an inflammatory disease successfully treated with TNF-α antibodies.(27) In addition, carrying the class II HLA-DRB1*0103 allele correlates with an increased risk for developing ulcerative colitis.(28)

Several studies have suggested that monocytes per se are targeted by conventional IBD therapy.(6, 29, 30) Our results suggest that specific down-regulation of the HLA-DRhi subpopulation may be an important mechanism behind resolution of the inflammation. In this study, patients treated with Granulocyte/Monocyte apheresis were added for comparison since Adacolumn® is the only treatment specifically targeting circulating monocytes. These cells are removed through Fcγ receptor binding to the cellulose acetate beads in the column, leaving circulating T-cells unaffected.(18) Corticosteroid therapy mediates a decrease in the number of circulating CD14+HLA-DRhi monocytes that is comparable to GMA (Figure 3B). This suppression seems to be important for the induction of remission, since significant decrease of CD14+HLA-DRhi monocytes is only observed in the patients achieving long-term remission (Figure 3D). Interestingly, patients subjected to biological treatment did not display a decrease of pro-inflammatory monocytes (Figure 3C). We speculate that by removing TNF-α, one of the main products of these monocytes, autocrine feed-back mechanisms leading to cellular activation might be induced. The observation underscores the difference in mode of action between anti-TNF-α antibodies and corticosteroids and should be further investigated since anti-TNF-α failure may partly depend on the monocytes' production of additional pro-inflammatory chemokines, cytokines and integrin receptors counterbalancing TNF-α suppression.

Classically, leukocyte populations have been defined through their capability to produce inflammatory mediators such as cytokines and chemokines. In order to gain a functional understanding of how CD14+HLA-DRhi monocytes mediate inflammation, we investigated their pro-inflammatory potential at the mRNA level compared to their HLA-DRlo-expressing counterpart. In this context, the HLA-DRhi subset produced markedly elevated levels of gene transcripts associated with activation and pro-inflammatory phenotype. The population displayed a 500-fold increase of TNF-α transcript levels, which establishes the HLA-DRhi subset as one of the most important producers of this cytokine. Other genes were also investigated by PCR array analysis, revealing the highest up-regulation in CCL4, CCL3 and IL-1β, all cytokines previously described to be involved in the recruitment of inflammatory cells to the intestinal mucosa in IBD (Figure 4D).(31-35) The most prominent difference was observed in the CCL4/MIP-1β gene, with up-regulated transcript levels of more than 300-fold. The inflammatory role of CCL4 was reported by Bystry and colleagues; activated T helper cells were demonstrated to efficiently migrate towards a CCL4 tissue gradient through CCR5 interaction.(31) Thus, our finding that CCL4 transcripts were produced in high levels by CD14+HLA-DRhi monocytes supports their inflammatory potential.(36) The transcript with the most apparent down-regulation in HLA-DRhi monocytes was the HDAC4 gene that encodes a histone deacetylase that functions as a transcriptional repressor.(19) Together, these data indicate that CD14+HLA-DRhi is a transcriptionally active subset that readily expresses genes important for mediating mucosal immune responses.

On the surface level, CD14+HLA-DRhi monocytes only partially express CD16, suggesting that the population constitutes a separate subset that is not included in its entirety when defining pro-inflammatory monocytes through their expression of CD1410 CD16+ (Figure 5C). The population is further defined by its expression of CCR7 and the gut-homing receptor CCR9, which clearly distinguishes HLA-DRhi monocytes from the CD14+CD16- and CD14loCD16+ subsets (Figure 5).

In the context of intestinal inflammation, interactions between CCR9-expressing T cells and CCL25 (TECK) expressed in the gut epithelium have been implicated as an important mechanism for recruiting circulating lymphocytes to the intestinal mucosa. (21) However, whether this mechanism also applies for the extensive infiltration of blood monocytes to the intestinal mucosa observed during inflammation has never been studied. (37) It was recently shown that CCR9-expressing monocytes are increased in peripheral blood of patients with rheumatoid arthritis. However, the role of these cells in IBD remains unclear. In accordance with the results from this study, we show that the CD14+CD16- and CD14loCD16+ subsets express similar levels of CCR9.(38) Those levels were notably superseded by the CCR9 expression on CD14+HLA-DRhi monocytes (Figure 5A). Surprisingly, this expression was considerably higher than that observed on T cells, which is considered to be the main CCR9-carrying cell type (Supplementary Figure 1A).(23) In contrast, the expression of CCR2, another chemokine receptor important for monocyte relocation in several disease groups, was not increased on HLA-DRhi monocytes (Figure 5B).(24) This suggests that the specific increase in CCR9 expression among CD14+HLA-DRhi may reflect a gut-specific phenotype, rather than a generally activated subset. The ligand for CCR9, CCL25, was found to be expressed in mucosal tissue by QT-PCR analysis, which is supported by other reports identifying CCL25 in the colon (Supplementary Figure 1B).(22, 39) In conclusion, these data suggest that monocytes in general and CD14+HLA-DRhi in particular possess the ability to relocate to the intestinal mucosa through CCR9-CCL25 interactions.

T cells have been shown to acquire their CCR9 expression through retinoic-acid dependent imprinting by mesenterial lymph node dendritic cells.(40) The issue of whether CCR9 expression on monocytes is acquired through similar mechanisms seems controversial, especially since blood monocytes are not known to traffic lymph nodes to the same extent as T cells. Here, we show that CD14+HLA-DRhi monocytes are defined by their high expression of CCR7, a marker mainly described as a lymph-node homing receptor for dendritic cells and T helper cells (Figure 5A).(20) Therefore, it is tempting to speculate that CCR7-expressing CD14+HLA-DRhi monocytes traffic the lymph nodes to a higher extent than has previously been known, and that CCR9 imprinting in these monocytes may occur through mechanisms similar to those reported in T cells. Being beyond the scope of this study, the mechanisms behind CCR9 induction in monocytes, as well as the functional role of their CCR7 expression, need to be addressed in the future.

In this study, we have shown that CD14+HLA-DRhi blood monocytes are increased in patients with active intestinal inflammation, and correlate with disease severity as defined by the UC-DAI and HBI disease activity indices. Furthermore, these monocytes produce major amounts of inflammatory mediators and express high levels of the gut-homing receptor CCR9. In summary, these findings indicate that CD14+HLA-DRhi blood monocytes play an important role in IBD and that future studies evaluating these monocytes as specific targets for IBD therapy are highly motivated.

### References

1. Bouma G, Strober W. The immunological and genetic basis of inflammatory bowel disease. Nat Rev Immunol. 2003;3:521-533
2. Desreumaux P, Brandt E, Gambiez L, et al. Distinct cytokine patterns in early and chronic ileal lesions of Crohn's disease. Gastroenterology. 1997;113:118-126
3. Hugot JP, Laurent-Puig P, Gower-Rousseau C, et al. Mapping of a susceptibility locus for Crohn's disease on chromosome 16. Nature. 1996;379:821-823
4. Hugot JP, Chamaillard M, Zouali H, et al. Association of NOD2 leucine-rich repeat variants with susceptibility to Crohn's disease. Nature. 2001;411:599-603
5. Arseneau KO, Tamagawa H, Pizarro TT, et al. Innate and adaptive immune responses related to IBD pathogenesis. Curr Gastroenterol Rep. 2007;9:508-512
6. Hanai H, lida T, Takeuchi K, et al. Adsorptive depletion of elevated proinflammatory CD14+CD16+DR++ monocytes in patients with inflammatory bowel disease. Am J Gastroenterol. 2008;103:1210-1216
7. Mahida YR, Wu KC, Jewell DP. Respiratory burst activity of intestinal macrophages in normal and inflammatory bowel disease. Gut. 1989;30:1362-1370
8. D'Haens G, Sandborn WJ, Feagan BG, et al. A review of activity indices and efficacy end points for clinical trials of medical therapy in adults with ulcerative colitis. Gastroenterology. 2007; 132:763-786
9. Rugtveit J, Brandtzaeg P, Halstensen TS, et al. Increased macrophage subset in inflammatory bowel disease: apparent recruitment from peripheral blood monocytes. Gut. 1994;35:669-674
10. Smythies LE, Maheshwari A, Clements R, et al. Mucosal IL-8 and TGF-beta recruit blood monocytes: evidence for cross-talk between the lamina propria stroma and myeloid cells. J Leukoc Biol. 2006;80:492-499
11. Feng G, Lu J, Gross J. Generation of transgenic mice. Methods Mol Med. 2004;99:255-267
12. Frankenberger M, Sternsdorf T, Pechumer H, et al. Differential cytokine expression in human blood monocyte subpopulations: a polymerase chain reaction analysis. Blood. 1996;87:373-377
13. Beige KU, Dayyani F, Horelt A, et al. The proinflammatory CD14+CD16+DR++ monocytes are a major source of TNF. J Immunol. 2002;168:3536-3542
14. Tallone T, Turconi G, Soldati G, et al. Heterogeneity of human monocytes: an optimized four-color flow cytometry protocol for analysis of monocyte subsets. J Cardiovasc Transl Res. 2011;4:211-219
15. Genel F, Atlihan F, Ozsu E, et al. Monocyte HLA-DR expression as predictor of poor outcome in neonates with late onset neonatal sepsis. J Infect. 2010;60:224-228
16. Fu Q, Cui N, Yu W, et al. Percentages of CD4+ T regulatory cells and HLA-DR expressing monocytes in severe intra-abdominal infections. Scand J Infect Dis. 2010;42:475-478
17. Blumberg RS, Saubermann LJ, Strober W. Animal models of mucosal inflammation and their relation to human inflammatory bowel disease. Curr Opin Immunol. 1999; 11:648-656
18. Saniabadi AR, Hanai H, Takeuchi K, et al. Adacolumn, an adsorptive carrier based granulocyte and monocyte apheresis device for the treatment of inflammatory and refractory diseases associated with leukocytes. Ther Apher Dial. 2003;7:48-59
19. Bertos NR, Wang AH, Yang XJ. Class II histone deacetylases: structure, function, and regulation. Biochem Cell Biol. 2001;79:243-252
20. Forster R, Davalos-Misslitz AC, Rot A. CCR7 and its ligands: balancing immunity and tolerance. Nat Rev Immunol. 2008;8:362-371
21. Johansson-Lindbom B, Agace WW. Generation of gut-homing T cells and their localization to the small intestinal mucosa. Immunol Rev. 2007;215:226-242
22. Wurbel MA, Philippe JM, Nguyen C, et al. The chemokine TECK is expressed by thymic and intestinal epithelial cells and attracts double- and single-positive thymocytes expressing the TECK receptor CCR9. Eur J Immunol. 2000;30:262-271
23. Zabel BA, Agace WW, Campbell JJ, et al. Human G protein-coupled receptor GPR-9-6/CC chemokine receptor 9 is selectively expressed on intestinal homing T lymphocytes, mucosal lymphocytes, and thymocytes and is required for thymus-expressed chemokine-mediated chemotaxis. J Exp Med. 1999;190:1241-1256
24. Deshmane SL, Kremlev S, Amini S, et al. Monocyte chemoattractant protein-1 (MCP-1): an overview. J Interferon Cytokine Res. 2009;29:313-326
25. Don Porto Carero A, Hoet PH, Nemery B, et al. Increased HLA-DR expression after exposure of human monocytic cells to air particulates. Clin Exp Allergy. 2002;32:296-300
26. Portillo G, Turner M, Chantry D, et al. Effect of cytokines on HLA-DR and IL-1 production by a monocytic tumour, THP-1. Immunology. 1989;66:170-175
27. Ridley MG, Kingsley G, Pitzalis C, et al. Monocyte activation in rheumatoid arthritis: evidence for in situ activation and differentiation in joints. Br J Rheumatol. 1990;29:84-88
28. Anderson CA, Massey DC, Barrett JC, et al. Investigation of Crohn's disease risk loci in ulcerative colitis further defines their molecular relationship. Gastroenterology. 2009; 136:523-529 e523
29. Fingerle-Rowson G, Angstwurm M, Andreesen R, et al. Selective depletion of CD14+ CD16+ monocytes by glucocorticoid therapy. Clin Exp Immunol. 1998;112:501-506
30. Koch S, Kucharzik T, Heidemann J, et al. Investigating the role of proinflammatory CD16+ monocytes in the pathogenesis of inflammatory bowel disease. Clin Exp Immunol.161:332-341
31. Bystry RS, Aluvihare V, Welch KA, et al. B cells and professional APCs recruit regulatory T cells via CCL4. Nat Immunol. 2001;2:1126-1132
32. Ottonello L, Montecucco F, Bertolotto M, et al. CCL3 (MIP-1alpha) induces in vitro migration of GM-CSF-primed human neutrophils via CCR5-dependent activation of ERK 1/2. Cell Signal. 2005;17:355-363
33. Ramos CD, Canetti C, Souto JT, et al. MIP-1alpha[CCL3] acting on the CCR1 receptor mediates neutrophil migration in immune inflammation via sequential release of TNF-alpha and LTB4. J Leukoc Biol. 2005;78:167-177
34. Uguccioni M, Gionchetti P, Robbiani DF, et al. Increased expression of IP-10, IL-8, MCP-1, and MCP-3 in ulcerative colitis. Am J Pathol. 1999;155:331-336
35. Grimm MC, Elsbury SK, Pavli P, et al. Enhanced expression and production of monocyte chemoattractant protein-1 in inflammatory bowel disease mucosa. J Leukoc Biol. 1996;59:804-812
36. Zhou L, Braat H, Faber KN, et al. Monocytes and their pathophysiological role in Crohn's disease. Cell Mol Life Sci. 2009;66:192-202
37. Grimm MC, Pullman WE, Bennett GM, et al. Direct evidence of monocyte recruitment to inflammatory bowel disease mucosa. J Gastroenterol Hepatol. 1995;10:387-395
38. Schmutz C, Cartwright A, Williams H, et al. Monocytes/macrophages express chemokine receptor CCR9 in rheumatoid arthritis and CCL25 stimulates their differentiation. Arthritis Res Ther. 2010;12:R161
39. Walters M, Berahovich R, Wang Y, et al. Presence of CCR9 and its ligand CCL25/TECK in the colon: Scientific rationale for the use of CCR9 small molecule antagonist CCX282-B in colonic disorders [abstract]. Gut2008:OP184
40. Iwata M, Hirakiyama A, Eshima Y, et al. Retinoic acid imprints gut-homing specificity on T cells. Immunity. 2004;21:527-538
41. Vermeire S, Schreiber S, Sandborn WJ, et al. Correlation between the Crohn's Disease Activity and Harvey-Bradshaw Indices in Assessing Crohn's Disease Severity. Clin Gastroenterol Hepatol.

### Tables

**Table 1. Patient demography.**

| | | |
|---|---|---|
| **Gender** | Male | 32 |
| | Female | 19 |
| **Age mean** | | 37.9 |
| **Diagnosis** | Ulcerative colitis | 31 |
| | Crohn's disease | 20 |
| **Extension** | Extensive | 25 |
| | Left-sided | 19 |
| | Proctitis | 5 |
| | Ileocekal | 2 |
| **Intervention** | Corticosteroids^{1,2} | 16 |
| | Anti-TNF-α^{3,5} | 17 |
| | GMA apheresis^{4,5} | 18 |
| **Azathioprine** | Yes | 21 |
| | No | 30 |

| | | |
|---|---|---|
| 1. Fifteen patients were introduced to 20-45 mg prednisone followed by tapering of 5 mg weekly. 2. One patient received topical corticosteroids for ulcerative proctosigmoiditis. 3. Anti-TNF-α treatment was administered either as infusions of 5 mg/kg infliximab week 0, 2 and 6 or subcutaneous injections of 80 mg Adalimumab week 0 followed by 40 mg every other week. 4. In the GMA apheresis group, each patient received a total of 5-8 Adacolumn® leukocytapheresis session 1-2 times weekly. 5. Some patients were receiving baseline corticosteroid medication | | |

**Table 2. Flow cytometry antibodies used in the study**

| **Marker** | **Conjugate** | **Clone** | **Manufacturer** |
|---|---|---|---|
| CD4 | Pacific Blue | RPA-T4 | BD |
| CD14 | APC | M5E2 | BD |
| CD16 | PE-Cy7 | 3G8 | BD |
| HLA-DR | PerCP | L243 | BD |
| CCR1 | Alexa Fluor 647 | TG4 | Biolegend |
| CCR2 | PerCP-Cy5.5 | TG5 | Biolegend |
| CCR3 | PE | 5E8 | Biolegend |
| CCR5 | PE | HM-CCR5 | Biolegend |
| CCR6 | PerCP-Cy5.5 | 11A9 | BD |
| CCR7 | PerCP-Cy5.5 | TG8 | Biolegend |
| CCR9 | APC | 112509 | R&D Systems |
| CCR10 | PE | 314305 | R&D Systems |
| CXCR1 | APC | 8F1 | Biolegend |
| CXCR5 | PerCP-Cy5.5 | TG2 | Biolegend |
| CXCR6 | PE | 56811 | R&D Systems |

### REFERENCE EXAMPLE 2 - Affinity of blood cells to CCL25.

### Materials and methods

Isolation of peripheral blood leukocytes. Heparinized peripheral blood from healthy blood donors or IBD patients was fixed with 4% paraformaldehyde for 4 minutes, hemolyzed for 15 minutes with a 0.83 % ammonium chloride solution and washed twice in FACS buffer to obtain a suspension of blood leukocytes.

Chemokines. The leukocytes were incubated for 30 min in the dark at 4 °C with the following biotinylated and Alexa647 Fluor® labeled chemokines: CCL25 (in concentrations of 0,1 ng/µL, 0,5 ng/µL and 5 ng/µL). The cells were then washed with FACS-buffer and analyzed by flow cytometry. All chemokines used in the Examples were provided by Almac Sciences Scotland Ltd, Edinburgh, Scotland.

Flow cytometry assay. The flow cytometry assay was performed on a two laser FACS Calibur cytometer (BD Immunocytometry systems, San Jose, Ca, USA). Ten thousand cells were counted and analysed in each sample. For data analyses, Cell Quest Pro software from Becton Dickinson was used.

In the experiment with biotinylated CCL25 it was found that neither T-cells (CD4+ lymphocytes; CD8+ lymphocytes) nor monocytes (CD14+ monocytes) from the peripheral blood of a healthy donor (Fig. 7a, 7b and 7c) bound to the biotinylated chemokine. In contrast, about 80% of the CD8+ lymphocytes and about 90% of the CD4+ lymphocytes and the monocytes from a patient with Crohn's disease bound to CCL25 (Fig 8a, 8b and 8c).

**REFERENCE EXAMPLE 3** - Preparation of a chemokine column for blood cell apheresis. To streptavidin cross-linked agarose (ProZyme, San Leandro, CA, U.S.A.) beads in the range from 75 µm to 300 µ suspended (200 ml, ∼50 %, v/v) in an aqueous solution of 25 mM sodium phosphate (pH 7.0) and 150 mM NaCl was added a solution of 75 µg biotinylated CCL25 (Almac Sciences) in the same buffer at 22 °C and slowly stirred by hand for 3 min. After standing for another 20 min, the support was filtered off, washed thrice with neutral aqueous sodium phosphate/sodium chloride and filled into a glass column (i.d. 25 mm, length 12 cm).

**REFRERENCE EXAMPLE 4** - Separation of monocytes from peripheral blood of a healthy donor with the chemokine column of Example 3. Heparinized peripheral blood from a healthy male donor was analyzed by flow cytometry for CD4+ lymphocytes, CD8+ lymphocytes and CD14 monocytes. 100 ml of the blood was filtered through the column at a rate of about 8 ml per min and washed with FACS buffer. The filtered blood was analyzed for the same cells. It was found that about 95 % of the monocytes had been retained by the column whereas more than 90 % each of CD4+ and CD8+ lymphocytes had been recovered.

### REFERENCE EXAMPLE 5 - Tailored leukapheresis

### COLUMN DESIGN AND PROPERTIES

### Introduction

Apheresis is an established treatment used for depletion of blood components, such as antibodies, low-density lipoproteins (LDL) and blood cells. Leukapheresis is the apheresis treatment used for removal of white blood cells, leukocytes. The patient is connected to an extracorporeal blood circulating system; the blood is drawn from a vein in one arm, passed through a column device and returned into the other arm of the patient. Side effects of leukapheresis treatments are varying from mild events like headache, dizziness, hypotension, palpitation and flush seen in 0.1 to 5% of treated patients.

### The column

The column is intended to be used as a leukapheresis treatment for inflammatory bowel disease, including Crohn's disease (CD) and ulcerative cholitis (UC). It will specifically remove CD14, HLA-DR, CCR9 and/or CCR7-expressing gut-homing leukocytes through the use of suitable binding reagents contained on a resin, exploiting the CD14, HLA-DR, CCR9 and/or CCR7-binding reagent interaction. The column consists of three combined components, the plastic house, the streptavidin (SA) Sepharose™ BigBeads matrix and binding reagent bound to the matrix. The treatment is conducted using the same techniques as a standard apheresis procedure.

### The plastic house (FIG. 9)

The plastic house, designed to keep a continuous blood flow through the matrix, consists of a transparent body and red-coloured top. The top has a distribution plate (2) at the inflow site (1) to spread the blood evenly over the entire matrix area. The plate is the first safety barrier preventing larger particles flowing through the column and into the patient. Safety filter units (3 and 4) are placed at the inflow (1) and outflow (5) sites of the plastic housing. The safety filter unit contains three filters designed to be a robust barrier and stop all particles larger than blood cells passing through the column. The plastic housing design is shown in Figure 9. The design with safety filters (3 and 4) at both ends of the column device will minimize the risk of leakage of particles into the patient, including in the event that the device is placed up side down with the blood flow in the opposite direction to that anticipated.

### Streptavidin Sepharose™ BigBeads

The second component in the device is the affinity matrix called streptavidin Sepharose™ BigBeads (Sepharose™ GE Healthcare, Sweden). Sepharose™ is a cross linked, beaded-form of agarose, which is a polysaccharide extracted from seaweed. Sepharose™ and agarose are commonly used as column matrices in biomedical affinity techniques. It is chosen for its optimal distribution capacity and can provide a large available area for affinity binding.

### bTECK

Coupled to the matrix is the third component of the device, in this example the bTECK. This bTECK peptide is a synthetic, engineered version of the human chemokine TECK, which is truncated and biotinylated, but retains its binding activity to the TECK receptor CCR9. By biotinylating the engineered TECK, it is able to bind to the streptavidin molecules in the Sepharose™ matrix. The biotin-streptavidin binding is known be one of the strongest biological interactions with a Kd in the order of 4 x 10-14 M. The calculated ratio of streptavidin:biotin binding sites in the column is 10:1. Therefore, the coupling between the matrix and bTECK will be immediate, minimising the risk of bTECK decoupling from the matrix.

### The apheresis system

To conduct the leukapheresis the following components are needed; the column, tubing system, and a 4008 ADS pump (Fresenius Medical Care).

### The circuit

The system is illustrated in Figure 10. The patient (1) is connected to the extracorporeal circuit via sterile Venflon needles to veins in the right and the left arms. A saline bag (3) is also connected and the saline solution is pumped with an ACD pump (2). Blood is drawn from one arm of the patient through the sterile tubing system by the blood pump (4) and passed through the column (6) and back to the patient. The tubing system is connected to the column via standard dialysis luer-lock couplings. The couplings on the column are colour-coded for correct assembly; red tubing for inflow to the red column top and blue tubing for outflow back to the patient. An air detector (8) is present. Inlet pressure(5) and Pven sensors (7) are employed to monitor the pressure in the circuit.

### The 4008 ADS pump

An apheresis pump, from Fresenius Medical Care, monitors the patient's inflow and outflow, the pressure in the extracorporeal circulation and can discriminate air by a bubble catcher and air detector. A clot catcher filter is placed inside the bubble catcher. The pump also has an optical detector to distinguish between light, e.g. saline solution or air present in the tubing system and dark e.g. blood present in the tubing system.

A schematic diagram of the pump, showing the air detector and optical filter is shown in Figure 11. If the pump system detects air bubbles and optical fluctuations or if extracorporeal pressure values are out of the set range, then the pump stops immediately and a visual/ audible alarm are emitted.

Legend for FIG. 11:
1. Monitor
2. Holder for waste bag
3. Modules (left to right - Blood pump, ACD pump, Air detector)
4. Reserve places for further modules
5. Absorber holder
6. Drip detector
7. IV pole

### Preparation of the patient

The patient will be administered anticoagulants prior to each treatment session. A sterile saline solution with 5000 IE Heparin will be used for priming the extracorporeal system, thereafter a bolus injection with 4000 IE Heparin will be added into the circuit at the start of each treatment session.

### Leukapheresis time and flow rate

The apheresis system should be operated at a flow rate of 30-60 mL/min. A treatment is finalised after 1800mL of blood has been circulated.

### Storage conditions

The column devices should be stored between 1 and 25°C avoiding freezing and more elevated temperatures. Stability data > 3 months indicate no difference in functionality over time or by temperature (room temperature and refrigerated). The columns will be kept in refrigerated conditions until use. Mechanical damage as those resulting from violent vibrations and trauma should be avoided. Column stored outside of these recommendations should not be used.

### Transport conditions

The column devices will be transported under refrigerated condition, avoiding freezing and more elevated temperatures. Mechanical damage such as those resulting from violent vibrations and trauma should be avoided.

### REFERENCE EXAMPLE 6 - NON-CLINICAL STUDIES

### In-vitro depletion of target cell populations

To investigate the ability to eliminate CCR9-expressing cells, in vitro tests have been performed on the bTECK coupled matrix. Blood was collected from blood donors and inflammatory bowel disease patients and passed through the column device containing bTECK coupled matrix. Blood samples were taken before and after column passage and analyzed by flow cytometry (FACS) for the depletion of CCR9-expressing cells.

The results demonstrate significant depletion of the target population CD14- positive CCR9-expressing cells post matrix perfusion; while total CD14-positive cells remain unchanged. Depletion tests were performed on blood from healthy donors and IBD patients confirming similar effects. The results are shown in Figures 12 and 13 respectively.

In conclusion, the in-vitro results demonstrate a specific reduction of 50-75% of the CCR9-expressing cells by the column. Non-CCR9-expressing cells remained unaffected.

### REFERENCE EXAMPLE 7 - TECK-PEG-Biotin Synthesis Summary

### Target molecule:

### TECK (Met to Nleu substitution) derivatised at the ε-amino side chain functionality of Lys72 with PEG-Biotin (TFA salt)

### Modifications:

Truncated form of human TECK corresponding to residues 1-74 of the mature protein, which encompasses the sequence corresponding to the chemokine fold. The full length mature protein is 127 amino acids (the signal peptide is 23 amino acids in a 150 amino acid immature protein). The single methionine within the sequence was altered to Norleucine, to mitigate against oxidation of this residue during the chain assembly, which was observed during the synthesis of the natural sequence derivative. The Gln at the N-terminus of the proteins is subject to pyroGlu formation under physiological conditions. Thus Gln1 of the sequence was substituted with pyroglutamine to prevent mixed species of N-terminal Gln and pyroGlu being generated. This improves the yield of synthesis and ensures a homogeneous chemokine preparation through column manufacture and use. The naturally occurring lysine at position 72 was modified through biotinylation on the resin. A PEG spacer was incorporated between the ε-amino functionality and the biotin.

The linear amino acid sequence (SEQ ID NO: 1) is shown, prior to attachment of the PEG spacer and biotin molecules at amino acid 72 (K): H-XGVFEDCCLAYHYPIGWAVLRRAWTYRIQEVSGSCNLPAAIFYLPKRHRKVCGNPKSREVQ RAXKLLDARNKVF-OH
X1 = pyroGlu or Gln
X64 = Norleucine

The engineered TECK sequence was assembled on a solid support, using Fmoc protocols for solid-phase peptide synthesis (SEQ ID NO: 2):
H-XGVFEDCCLAYHYPIGWAVLRRAWTYRIQEVSGSCNLPAAIFYLPKRHRKVCGNPKSREVQ RAXKLLDARNXVF-RESIN
X1 = pyroGlu or Gln
X64 = Norleucine
X72 = K(Dde)
FmocLys(Dde)-OH was incorporated as residue 72 to facilitate site-specific labelling at this position of the protein.
Met to Nle substitution.
N-terminal Gln to pyroglutamic acid substitution.

### Removal of Dde Protection:

The Dde protecting group was removed by treatment of all resin (2.5g) with a solution of 2% hydrazine in DMF (100ml) over 1 hour period to afford 2.0g resin.

### Labelling steps:

### 1. Couple Fmoc-8-amino-3,6-dioctanoic acid

Resin (1.5g) was swollen in DMF (2ml) and then a solution of Fmoc-8-amino-3,6-dioctanoic acid (0.38g, 1mmol), DIC solution (2ml, 0.5M in DMF) and HOCt solution (2ml, 0.5M in DMF) was added. The mixture was sonicated for 2 hours and then washed with DMF.

### 2. Cap

The resin was capped with 0.5M acetic anhydride/DMF solution (20ml) for 5 minutes and then washed with DMF.

### 3. Fmoc deprotection

Fmoc deprotection was carried out by treatment with 20% piperidine in DMF solution (2 x 50ml) for 15 minutes each. The resin was washed with DMF.

### 4. Couple Biotin-OSu

A solution of Biotin-NHS ester (341mg, 1mmol) and DIPEA (348ul, 2mmol) in DMF (10ml) was added to the resin and the mixture was sonicated for 3 hours. The resin was washed thoroughly with DMF and DCM then dried in vacuo. Dry resin obtained = 1.5g.

### Cleavage:

Dry peptide resin (1.5g) and the mixture was cleaved with TFA (30 ml) containing a scavenger cocktail consisting of TIS, thioanisole, water, EDT and phenol and the mixture was stirred at room temperature for 6 hours. The solution was filtered into cold ether and the resin rinsed with TFA. The peptide was centrifuged, washed with ether, centrifuged and lyophilised to give 1.0g crude peptide.

### Folding Protocol:

Crude peptide (100mg) was dissolved into 6M GnHCI (233ml) and then rapidly diluted to 2M GnHCI concentration by the addition of 50mM TRIS pH8 (467ml) containing 0.5mM GSSG and 5mM GSH. The mixture was stirred at room temperature for 2.5 days and then analysed by HPLC (Jupiter C18, 250x4.6mm column, 10-60% B over 30 minutes. HPLC analysis confirmed the formation of desired product as well as mis-folded by-products.

### Purification:

The folded protein was purified by reverse phase HPLC using a Jupiter C18, 250x21mm column, 9ml/min, 10-60%B over 50 minutes. 11.1mg of pure folded Nle-TECK-Biotin was afforded.

Figure 14 shows HPLC of purified folded Biotin-TECK(Nleu). The protein eluted in a single peak at 21.6 mins.

Figure 15 shows Electrospray ionisation with tandem mass spectrometry (ES/MS) data of purified folded Biotin-TECK(Nleu). The expected mass was 8959.4 Da.

### Functional Assay Data:

TECK-Biotin-Nle was tested for agonist activity in an Aequorin assay against hCCR9, (Euroscreen) and an EC50 value of 63.6nM was reported. c.f. EC50 for native TECK is 67.87nM.

The final active chemokine thus has the following sequence (SEQ ID NO: 3):
H-XGVFEDCCLAYHYPIGWAVLRRAWTYRIQEVSGSCNLPAAIFYLPKRHRKVCG
NPKSREVQRAXKLLDARNXVF-OH
X1 = pyroGlu
X64 = norleucine
X72 = an amino acid residue that can be biotinylated, such as lysine, ornithine or diaminopropionic acid and optionally is biotinylated, optionally via a spacer molecule such as PEG, such as K(PEG-Biotin)

### REFERENCE EXAMPLE 8 - Synthesis of biotinMIP-3β (CCL19)

### Assembly:

Chemical synthesis of chemokines was performed using standard Fmoc solid phase peptides synthesis (SPPS) techniques on an ABI 433 peptide synthesiser. DIC (0.5 M in DMF) and OxymaPure (0.5 M in DMF) were used for activation, acetic anhydride (0.5 M in DMF) for capping, and 20 % piperidine in DMF for Fmoc deprotection. Rink Amide resin was utilised for the generation of C-terminal amide chemokines and Wang resin for C-terminal acid chemokines. After assembly, the resin was washed with DMF and DCM and then dried *in vacuo.*

### Removal of Dde Protection:

The Dde protecting group was removed by treatment of resin with a solution of 2.5% hydrazine in DMF (200ml) over a 2 hour period. The resin was then washed with DMF.

### Labelling steps:

### 1. Couple Fmoc-8-amino-3,6-dioctanoic acid (PEG)

Resin was swollen in DMF and then a solution of Fmoc-8-amino-3,6-dioctanoic acid (0.38g, 1mmol), DIC solution (2ml, 0.5 M in DMF) and OxymaPure solution (2ml, 0.5 M in DMF) was added. The mixture was sonicated for 3 hours and then washed with DMF.

### 2. Capping

The resin was capped with acetic anhydride solution (0.5 M in DMF, 10ml) for 5 minutes and then washed with DMF.

### 3. Fmoc deprotection

Fmoc deprotection was carried out by treatment with 20% piperidine in DMF solution (2 x 50ml) for 15 minutes each. The resin was washed with DMF.

### 4. Couple Biotin-OSu

A solution of Biotin-OSu (341mg, 1mmol) and DIPEA (348µl 2mmol) in DMF (10ml) was added to the resin and the mixture was sonicated for 3 hours. The resin was washed thoroughly with DMF and DCM then dried *in vacuo.*

### Cleavage:

Dry resin was treated with TFA (10 ml) containing a scavenger cocktail consisting of TIS (500 µl), thioanisole (500 µl), water (500 µl), DMS (500 µl), EDT (250 µl), NH₄I (500 µg) and phenol (500 µg) and the mixture was stirred at room temperature for 5 hours. The solution was filtered into cold ether and the resin rinsed with TFA. The precipitated peptide was centrifuged, washed with ether, centrifuged and lyophilised.

### Purification Protocol:

The crude peptide was purified by reverse phase HPLC (RP-HPLC) using a Jupiter C18, 250 x 21 mm column, 9 ml/min, eluting with an optimised gradient [Buffer A: water containing 0.1% TFA, Buffer B: acetonitrile containing 0.1% TFA].

### Folding Protocol:

Pure peptide (10mg) was dissolved into 6M GnHCI (16 ml) and then rapidly diluted to 2M GnHCI concentration by the addition of 50mM TRIS pH 8.5 (84 ml) containing 0.3mM GSSG and 3mM GSH. The mixture was stirred at room temperature for 24 hours and then analysed by RP-HPLC (Jupiter C18, 250 x 4.6 mm column, 10-60% B over 30 minutes. Purification by RP-HPLC using an optimised gradient afforded the desired product.

### Target Molecule: MIP-3β derivatised at the ε-amino side chain functionality of Lys(78) with Biotin (TFA salt)

Modifications: Human MIP-3β corresponding to residues 1-77, is initially expressed as 98 amino acids comprising the chemokine fold, and a 21 amino acid signal peptide which is cleaved off. An additional lysine was inserted at the C-terminus, at position 78, and modified through biotinylation on the resin.

The linear amino acid sequence (SEQ ID NO: 4) is shown, prior to attachment of the biotin molecule at amino acid 78 (K):
H-GTNDAEDCCLSVTQKPIPGYIVRNFHYLLIKDGCRVPAVVFTTLRGRQLCA
PPDQPWVERIIQRLQRTSAKMKRRSSX-NH₂
X is an amino acid residue that can be biotinylated, such as lysine, ornithine or diaminopropionic acid and optionally is biotinylated, optionally via a spacer molecule such as PEG, e.g. K(PEG-Biotin)

The engineered MIP-3β sequence was assembled on a solid support (Rink Amide resin), using Fmoc protocols for solid-phase peptide synthesis as described in the general protocols section:
H-GTNDAEDCCLSVTQKPIPGYIVRNFHYLLIKDGCRVPAVVFTTLRGRQLCA
PPDQPWVERIIQRLQRTSAKMKRRSSX-RESIN
X is FmocLys(ivDde)

FmocLys(ivDde)-OH was incorporated as residue 78 to facilitate site-specific labelling at this position of the protein (SEQ ID NO: 5). Subsequent removal of the ivDde protecting group, followed by coupling of the Biotin, was carried out as described in the general protocol section. Cleavage, purification and folding protocols were carried out as described to furnish the desired active chemokine (SEQ ID NO: 6).
H-GTNDAEDCCLSVTQKPIPGYIVRNFHYLLIKDGCRVPAVVFTTLRGRQLCA
PPDQPWVERIIQRLQRTSAKMKRRSSX-NH₂
X is K(Biotin)

Electrospray ionisation with tandem mass spectrometry (ESI-TOF-MS) data of purified folded biotinMIP-3β: obtained = 9148.8 Da; expected 9149.7 Da.

### Functional Assay Data:

biotinMip-3β was tested for agonist activity in an Aequorin assay against hCCR7, (Euroscreen) and an EC50 value of 11.0nM was reported. c.f. EC50 for recombinant native MIP-3β is 1.6nM.

### REFERENCE EXAMPLE 9 Treatment of Crohn's Disease (CD)

### Materials and methods

### 1. Flow cytometric analysis of peripheral blood

Peripheral blood from patients and healthy controls was collected in heparin tubes. The red blood cells were lysed using Fix Buffer (Phosphate Buffer Saline (PBS) citrate with 4% paraformaldehyde) for four minutes at 37°C and Lysing buffer (PBS with 10mM Tris and 160mM NH₄Cl, pH 7.5) for 15 min at 37°C. The cells were washed in PBS with 2% Bovine Growth Serum, incubated with 10% human serum for 15min at room temperature (RT) and stained with antibodies (Table 2) at 4°C for 30min. The cells were analysed by flow cytometry on a FACS Canto flow cytometer with the FACSDiva software (BD Biosciences).

**Table 2. List of antibodies for flow cytometric analysis.**

| **Antibody** | **Fluorophore** | **Supplier** |
|---|---|---|
| CD14 | FITC | Beckman Coulter |
| Streptavidin | PE, APC | Biolegend |
| CD16 | PE Cy7 | BD Biosciences |
| CCR9 | APC | R&D Systems |
| HLADR | APC Cy7 | Biolegend |
| CD3 | V450 | BD Biosciences |
| CD19 | V500 | BD Biosciences |

### 2. Chemokine Binding test

Peripheral blood from patients and healthy controls was collected in heparin tubes. The red blood cells were lysed using Fix Buffer (Phosphate Buffer Saline (PBS) citrate with 4% paraformaldehyde) for four minutes at 37°C and Lysing buffer (PBS with 10mM Tris and 160mM NH4CI, pH 7.5) for 15 min at 37°C. The cells were washed in PBS with 2% Bovine Growth Serum, incubated with 10% human serum 15min at room temperature (RT) and stained with cell specific antibodies together with biotinylated chemokine (1 µM) or the corresponding chemokine receptor antibody at 4°C for 30min (Table 2). The biotinylated chemokine was detected via the interaction between biotin and a fluorophore conjugated Streptavidin. The samples were analysed by flow cytometry on a FACS Canto flow cytometer with the FACSDiva software (BD Biosciences).

### 3. Cell depletion by matrix conjugated with biotinylated chemokine

Cells were prepared from peripheral blood (section 1). 1 mL Sepharose BigBeads matrix conjugated with 0.4mg/mL Streptavidin (GE Healthcare) was washed in 50 mL PBS and added to a 5 mL polystyrene tube (BD Falcon™). Biotinylated chemokine (1µM) was added to the tube and incubated for 20 min at RT to enable immobilization of the chemokine on the matrix via the biotin-streptavidin interaction. Next, the cells were added to the chemokine-matrix and incubated for 20 min at RT. The cells that did not bind to the matrix were removed by washing the matrix with PBS in a sterile 40um nylon filter (BD Falcon™ Cell Strainer). The flow through cells were stained with antibodies (Table 2), analysed by flow cytometry and compared with cells from peripheral blood that had not been incubated with the chemokine-matrix.

### Results and Discussion

### Crohn's Disease (CD)

### 1. Flow cytometric analysis of peripheral blood

White blood cells from CD patients was analysed with flow cytometry. The patients exhibited increased numbers of circulating CCR9 expressing monocytes, a mean of 13% compared to approximately 7% in healthy blood (Fig 17).

### 2. Chemokine Binding test

CCR9 binds to the gut homing chemokine TECK (CCL25) and is important for cell migration to the small intestine. In accordance with the CCR9 expression, 14% of the monocytes bind to the biotinylated TECK (bTECK) (Fig 18).

### 3. Cell depletion by matrix conjugated with biotinylated chemokine

89% of the CCR9 expressing monocytes were efficiently depleted with bTECK-conjugated Sepharose Streptavidin Matrix. Before depletion there were 7.2% CCR9 expressing monocytes and after depletion 0.8% (Fig 19).

80% of the CCR9 expressing monocytes also have a high expression of HLADR suggesting a proinflammatory phenotype (Fig.20).

We conclude that monocytes in CD (CD14⁺HLA-DR^{hi} monocytes) express CCR9 and bind the ligand bTECK. Furthermore, the majority of the CCR9 expressing monocytes can be removed with Sepharose Streptavidin matrix conjugated with bTECK.

### REFERENCE EXAMPLE 10 Diagnosis and Treatment of IBS

The inventors have surprisingly found that Irritable Bowel Syndrome (IBS) patients, or subject suffering from IBS, display an increased frequency (or level) of chemokine receptor expressing cells, in particular monocytes, more specifically CCR9 expressing monocytes. Thus, IBS patients may display inflammation that is comparable to that shown by patients suffering from IBD. Irritable bowel syndrome (IBS) is a condition characterized by chronic abdominal pain, discomfort, bloating, and alteration of bowel habits. It is currently diagnosed on the basis of symptoms only. Accordingly, identification of a pro-inflammatory component provides new avenues for treatment and diagnosis of this debilitating condition.

### Materials and methods

### 1. Flow cytometric analysis of peripheral blood

Peripheral blood from patients and healthy controls was collected in heparin tubes. The red blood cells were lysed using Fix Buffer (Phosphate Buffer Saline (PBS) citrate with 4% paraformaldehyde) for four minutes at 37°C and Lysing buffer (PBS with 10mM Tris and 160mM NH4CI, pH 7.5) for 15 min at 37°C. The cells were washed in PBS with 2% Bovine Growth Serum, incubated with 10% human serum for 15min at room temperature (RT) and stained with antibodies (Table 3) at 4°C for 30min. The cells were analysed by flow cytometry on a FACS Canto flow cytometer with the FACSDiva software (BD Biosciences).

**Table 3. List of antibodies for flow cytometric analysis.**

| **Antibody** | **Fluorophore** | **Supplier** |
|---|---|---|
| CD14 | FITC | Beckman Coulter |
| Streptavidin | PE, APC | Biolegend |
| CD16 | PE Cy7 | BD Biosciences |
| CCR9 | APC | R&D Systems |
| HLADR | APC Cy7 | Biolegend |
| CD3 | V450 | BD Biosciences |
| CD19 | V500 | BD Biosciences |

### 2. Chemokine Binding test

Peripheral blood from patients and healthy controls was collected in heparin tubes. The red blood cells were lysed using Fix Buffer (Phosphate Buffer Saline (PBS) citrate with 4% paraformaldehyde) for four minutes at 37°C and Lysing buffer (PBS with 10mM Tris and 160mM NH4CI, pH 7.5) for 15 min at 37°C. The cells were washed in PBS with 2% Bovine Growth Serum, incubated with 10% human serum 15min at room temperature (RT) and stained with cell specific antibodies together with biotinylated chemokine (1 µM) or the corresponding chemokine receptor antibody at 4°C for 30min (Table 3). The biotinylated chemokine was detected via the interaction between biotin and a fluorophore conjugated Streptavidin. The samples were analysed by flow cytometry on a FACS Canto flow cytometer with the FACSDiva software (BD Biosciences).

### 3. Cell depletion by matrix conjugated with biotinylated chemokine

Cells were prepared from peripheral blood (section 1). 1 mL Sepharose BigBeads matrix conjugated with 0.4mg/mL Streptavidin (GE Healthcare) was washed in 50 mL PBS and added to a 5 mL polystyrene tube (BD Falcon™). Biotinylated chemokine (1 µM) was added to the tube and incubated for 20 min at RT to enable immobilization of the chemokine on the matrix via the biotin-streptavidin interaction. Next, the cells were added to the chemokine-matrix and incubated for 20 min at RT. The cells that did not bind to the matrix were removed by washing the matrix with PBS in a sterile 40um nylon filter (BD Falcon™ Cell Strainer). The flow through cells were stained with antibodies (Table 3), analysed by flow cytometry and compared with cells from peripheral blood that had not been incubated with the chemokine-matrix.

### Results and Discussion

### Irritable bowel syndrome (IBS)

White blood cells from IBS patients was analysed with flow cytometry. The patients exhibited increased numbers of circulating CCR9 expressing monocytes compared to healthy blood (Figure 21).

Irritable bowel syndrome is a condition characterized by chronic abdominal pain, discomfort, bloating, and alteration of bowel habits. It is currently diagnosed on the basis of symptoms only. The symptoms of IBS are very similar to IBD, Crohns disease (CD) and Ulcerative Colitis (UC), which also show an increase in CCR9 expressing monocytes (Figure 24).

CCR9 binds to the gut homing chemokine TECK (CCL25) and is important for cell migration to the small intestine. The monocytes in IBS patient blood bind to the biotinylated TECK (bTECK) (Figure 22).

Depletion of CCR9 expressing monocytes in IBS patient using bTECK conjugated sepharose streptavidin matrix is shown in Figure 23.

About 25% of the CCR9 expressing monocytes also have a high expression of HLADR suggesting a proinflammatory phenotype (Figure 25a). These cells can be depleted using bTECK conjugated sepharose streptavidin matrix (Figure 25b).

We conclude that monocytes in IBS express CCR9 and bind the ligand bTECK. Furthermore, the majority of the CCR9 expressing monocytes can be removed with Sepharose Streptavidin matrix conjugated with bTECK.

## Claims

1. A binding reagent capable of specifically binding to a marker of CD14⁺HLA-DR^{hi} monocytes selected from CCR9 for use in the treatment of inflammatory bowel disease, wherein the binding reagent is immobilized on a solid support contained within an apheresis column, to which is applied peripheral blood from a patient thus removing CD14⁺HLA-DR^{hi} monocytes from the peripheral blood of the patient or subject, and wherein the binding agent is an anti-CCR9 antibody.

2. The binding reagent of claim 1, wherein the inflammatory bowel disease is ulcerative colitis or Crohn's disease.

3. A method of diagnosing, monitoring progression of, or monitoring treatment of inflammatory bowel disease comprising determining the levels of monocytes expressing CCR9 in a sample obtained from a subject, wherein high levels of monocytes expressing CCR9, or increased levels of monocytes expressing CCR9 compared to the levels of monocytes expressing CCR9 in a healthy subject, indicate the presence or progression of inflammatory bowel disease, and wherein the subject has been treated using a binding agent according to claim 1.

4. The method of claim 3 wherein the inflammatory bowel disease is ulcerative colitis or Crohn's disease.

5. The method of claim 3 or 4 wherein the sample is a peripheral blood sample.

6. The method of any of claims 3-5, wherein decreased levels of monocytes expressing CCR9 correlate with successful treatment.

## Patentansprüche

1. Bindungsreagenz, das in der Lage ist, spezifisch an einen Marker von CD14⁺HLA-DR^{hi}-Monozyten zu binden, ausgewählt aus CCR9 zur Verwendung bei der Behandlung von entzündlicher Darmerkrankung, wobei das Bindungsreagenz auf einem festen Träger immobilisiert ist, der in einer Apheresesäule enthalten ist, auf den peripheres Blut von einem Patienten aufgebracht wird, wodurch CD14⁺HLA-DR^{hi}-Monozyten aus dem peripheren Blut des Patienten oder Individuums entnommen werden, und wobei das Bindemittel ein Anti-CCR9-Antikörper ist.

2. Bindungsreagenz nach Anspruch 1, wobei die entzündliche Darmerkrankung eine ulzeröse Colitis oder Morbus Crohn ist.

3. Verfahren zum Diagnostizieren, Überwachen des Fortschreitens oder Überwachen der Behandlung von entzündlichen Darmerkrankungen, umfassend das Bestimmen der Konzentrationen von Monozyten, die CCR9 exprimieren, in einer Probe, die von einem Individuum erhalten wurde, wobei hohe Konzentrationen von Monozyten, die CCR9 exprimieren, oder erhöhte Konzentrationen von Monozyten, die CCR9 exprimieren, im Vergleich zu den Konzentrationen von Monozyten, die CCR9 exprimieren bei einem gesunden Individuum, das Vorhandensein oder Fortschreiten von entzündlichen Darmerkrankungen anzeigen, und wobei das Individuum mit einem Bindemittel nach Anspruch 1 behandelt wurde.

4. Verfahren nach Anspruch 3, wobei die entzündliche Darmerkrankung eine ulzeröse Colitis oder Morbus Crohn ist.

5. Verfahren nach Anspruch 3 oder 4, wobei die Probe eine periphere Blutprobe ist.

6. Verfahren nach einem der Ansprüche 3-5, wobei verminderte Konzentrationen von Monozyten, die CCR9 exprimieren, mit einer erfolgreichen Behandlung korrelieren.

## Revendications

1. Réactif de liaison capable de se lier spécifiquement à un marqueur de monocytes CD14⁺HLA-DR^{hi} choisis parmi CCR9 destiné à être utilisé pour le traitement d'une maladie intestinale inflammatoire, dans lequel le réactif de liaison est immobilisé sur un support solide contenu au sein d'une colonne aphérèse, à laquelle est appliqué du sang périphérique provenant d'un patient, éliminant ainsi des monocytes CD14⁺HLA-DR^{hi} du sang périphérique du patient ou sujet, et dans lequel l'agent de liaison est un anticorps anti-CCR9.

2. Réactif de liaison selon la revendication 1, dans lequel la maladie intestinale inflammatoire est la rectocolite hémorragique ou maladie de Crohn.

3. Procédé de diagnostic, de surveillance de la progression de ou de surveillance du traitement d'une maladie intestinale inflammatoire, comprenant une détermination des taux de monocytes exprimant CCR9 dans un échantillon obtenu chez un sujet, dans lequel des taux élevés de monocytes exprimant CCR9, ou des taux accrus de monocytes exprimant CCR9, comparés aux taux de monocytes exprimant CCR9 chez un sujet sain, indiquent la présence ou la progression d'une maladie intestinale inflammatoire, et dans lequel le sujet a été traité en utilisant un agent liant selon la revendication 1.

4. Procédé selon la revendication 3 dans lequel la maladie intestinale inflammatoire est une rectocolite hémorragique ou maladie de Crohn.

5. Procédé selon la revendication 3 ou 4, dans lequel l'échantillon est un échantillon de sang périphérique.

6. Procédé selon l'une quelconque des revendications 3 à 5, dans lequel des taux réduits de monocytes exprimant CCR9 sont en corrélation avec un traitement réussi.
